# EUROPEAN PATENT APPLICATION

(11) **EP 1 630 559 A2**
(43) Date of publication of application: **01.03.2006**
(21) Application number: 05008803.8
(22) Date of filing: 31.12.1998
(51) Int. Cl.: G01N 33/573, G01N 33/68, G01N 33/86, G01N 33/566, C12Q 1/48, C12N 9/12

(54) **PYK2 (RAFTK) and inflammation**

(30) Priority: 30.12.1998 US 114465 P
(62) Divisional of application: 98966125.1
(71) Applicant: Sugen, Inc., South San Francisco, CA 94080 (US)
(72) Inventor: Schlessinger, Joseph, New York NY 10011 (US); Okigaki, Mitsuhiko, New York NY 10016 (US); Gishizky, Mikhail, Menlo Park CA 94025 (US)
(74) Representative: Viering, Jentschura & Partner

(57) **Abstract**

The present invention relates generally to the fields of immunology and medicine, and more specifically to the field of cellular signal transduction. The present invention relates, *inter alia*, to methods for diagnosis, treatment, and identification of therapeutics for particular inflammation-related diseases or disorders characterized by an interaction between a PYK2 polypeptide and a natural binding partner.

### Annex to the application documents - subsequently filed sequences listing

SEQUENCE LISTING

<110> Sugen, Inc.

<120> Pyk2 (RAFTK) and Inflammation

<130> P201195

<140> EP05008803.8
<141> 1998-12-31

<150> US 60/114,465
<151> 1998-12-30

<160> 5

<170> PatentIn version 3.3

<210> 1
<211> 30
<212> DNA
<213> Artificial

<220>
<223> Oligonucleotide primer (sense) for Pyk2 PCR construct

<400> 1
cgggatcctc atcatccatc ctaggaaaga 30

<210> 2
<211> 30
<212> DNA
<213> Artificial

<220>
<223> Oligonucleotide primer (antisense) for Pyk2 PCR construct

<400> 2
cgggaattcg tcgtagtccc agcagcgggt 30

<210> 3
<211> 21
<212> DNA
<213> Artificial

<220>
<223> Oligonucleotide primer (sense) for Pyk2-HA PCR construct

<400> 3
cacaatgtct tcaaacgcca c 21

<210> 4
<211> 63
<212> DNA
<213> Artificial

<220>
<223> Oligonucleotide primer (antisense) for Pyk2-HA PCR construct

<400> 4
ggctctagat cacgatgcgt agtcagggac atcgtatggg ractctgcag gtgggtgggc 60

cag 63

<210>  5
<211>  31
<212>  DNA
<213>  Artificial

<220>
<223>  Mutagenesis oligonucleotide primer for kinase negative Pyk2
       mutant

<400>  5
caatgtagct gtcgcgacct gcaagaaaga c                                    31

## Description

### RELATED APPLICATIONS

The present application is related to U.S. applications Serial No. 08/357,642, by Lev and Schlessinger, Lyon & Lyon Docket No. 209/070, entitled "PYK2 related Products and Methods", filed December 15, 1994; Serial No. 08/460,626, by Lev and Schlessinger, Lyon & Lyon Docket No. 211/121, entitled "PYK2 related Products and Methods", filed June 2, 1995; and Serial No. 08/987,689 by Lev and Schlessinger, Lyon & Lyon Docket No. 230/110, entitled "PYK2 related Products and Methods", filed December 9, 1997, all of which are hereby incorporated by reference herein in their entirety including any drawings, figures, or tables.

### FIELD OF THE INVENTION

The present invention relates generally to the fields of immunology and medicine, and more specifically to the field of cellular signal transduction.

### BACKGROUND OF THE INVENTION

None of the following discussion of the background of the invention, which is provided solely to aid the reader in understanding the invention, is admitted to be or to describe prior art to the invention. Cellular signal transduction is a fundamental mechanism whereby external stimuli that regulate diverse cellular processes are relayed to the interior of cells. One of the key biochemical mechanisms of signal transduction involves the reversible phosphorylation of tyrosine residues on proteins. The phosphorylation state of a protein is modified through the reciprocal actions of tyrosine phosphatases (TPs) and tyrosine kinases (TKs), including receptor tyrosine kinases and non-receptor tyrosine kinases.

RTKs are composed of at least three domains: an extracellular ligand binding domain, a transmembrane domain and a cytoplasmic catalytic domain that can phosphorylate tyrosine residues. The intracellular, cytoplasmic, non-receptor protein tyrosine kinases do not contain a hydrophobic transmembrane domain or an extracellular domain and instead contain non-catalytic domains in addition to their catalytic kinase domains. Such non-catalytic domains include the SH2 domains and SH3 domains. The non-catalytic domains are thought to be important in the regulation of protein-protein interactions during signal transduction.

PYK-2, also referred to as RAFTK, is a non-receptor tyrosine kinase that is activated by binding of ligand to G-coupled protein receptors such as bradykinin and acetylcholine. PYK2 has a predicted molecular weight of 111 kD and contains five domains: (1) a relatively long N-terminal domain; (2) a kinase catalytic domain; (3) a proline rich domain; (4) another proline rich domain; and (5) a C-terminal domain.

PYK2 is expressed in various tissues, including neural tissues, hematopoietic cells, and in some tumor cell lines. PYK2 is believed to regulate the activity of potassium channels in response to neurotransmitter signaling. PYK2 may also regulate ion-channel function by tyrosine phosphorylation.

PYK2 is activated by extracellular signals that lead to calcium influx or calcium release from internal stores. PYK2 is phosphorylated on tyrosine residues in response to a variety of external stimuli. PYK2 may provide a link between G-protein coupled receptors and calcium influx and the MAP kinase signaling pathway, a pathway that relays signals from the cell surface to regulate transcriptional events in the nucleus.

In the PCT Publication WO 98/07870 (Avraham, et al.), the authors state that
"...RAFTK therapeutics which modulate RAFTK activity in B cells, T cells, and monocytes can be used to treat immune-mediated disorders and mediate both cell mediated and humoral immune responses.
Normal hematopoietic cells are dependent on growth factors for growth and differentiation and the loss of this growth factor dependence can lead to autonomous growth. The involvement of *RAFTK* in several growth factor signaling pathways indicates that misse[x] pression of *RAFTK* can lead to the development of cancers, and the present invention contemplates modulating RAFTK expression and/or activity to control aberrant cell growth. In a preferred embodiment *RAFTK* is modulated to treat cancers of hematopoietic cells. In another embodiment malignancy can be suppressed in certain cells e.g., leukemic cells, by modulating *RAFTK* to induce cellular differentiation...
...In one embodiment the RAFTK proteins of the present invention can modulate the differentiation or maturation of hematopoietic cells; the subject RAFTK polypeptides are capable of influencing both the differentiation and maturation of pluripotent stem cells and the proliferation of differentiated cells."

### SUMMARY OF THE INVENTION

The present invention relates, inter alia, to methods for identification of compounds useful to treat or prevent inflammation-related diseases or disorders characterized by an interaction between a PYK2 polypeptide and a natural binding partner. In addition, the present invention relates to methods for diagnosis and for treatment of inflammation-related diseases or disorders characterized by an interaction between a PYK2 polypeptide and a natural binding partner.

The current invention demonstrates for the first time in an *in vivo* mouse model and a cellular model the link between PYK2 and the inflammatory response. To demonstrate the role of PYK2 *in vivo,* knockout mice lacking the *pyk2* gene were created using molecular genetic techniques. The inflammatory response of the knockout mice was compared with the corresponding mice not containing a *pyk2* deletion using two *in vivo* models: an influenza virus model and a subcutaneous carageenen air pouch model. Experiments using both models are described in detail herein in the Detailed Description of the Invention.

The data from these experiments confirm the role for PYK2 in cytokine release and support the importance of PYK2 function in inflammation. These experiments indicate that treatments that inhibit the functioning of PYK2 will be useful to decrease excessive inflammatory responses, whereas treatments to enhance the functioning of PYK2 will be useful to augment inadequate immune responses.

In a first aspect, the invention features a method for identifying one or more potential compounds useful to treat or to prevent a disease or disorder, wherein said disease or disorder is characterized by a inflammatory response, wherein said inflammatory response is characterized by an abnormality in a signal transduction pathway, and wherein said signal transduction pathway includes an interaction between a PYK2 polypeptide and a natural binding partner, comprising assaying said one or more potential compounds for those able to modulate said interaction as an indication of a useful said compound.

By "identifying" is meant investigating for the presence or absence of a property. The process may include measuring or detecting various properties, including the level of signal transduction and the level of interaction between a PYK2 polypeptide and a natural binding partner.

The term "compound" preferably refers to a non-peptide organic molecule, and most preferably refers to a non-peptide synthetic organic molecule. The term "non-peptide molecule" refers to a compound that is not a polymer of amino acids. A non-peptide molecule preferably does not contain chemical moieties that hydrolyze in physiological conditions, e.g. a peptidomimetic. Alternatively, a non-peptide molecule may be a peptoid, or modified nucleic acid molecule. Examples of compounds are included in the Description of the Invention, herein. Preferably, such molecules have a molecular weight less than 3,000.

By "inflammatory response" is meant a protective response elicited by injury or destruction of tissues, which serves to destroy, dilute, or wall off (sequester) both the injurious agent and the injured tissue. Histologically, it involves a complex series of events, including dilatation of arterioles, capillaries, and venules, with increased permeability and blood flow; exudation of fluids, including plasma proteins; and leukocyte migration into the inflammatory focus. A pathologic inflammatory response may be a continuation of an acute form or a prolonged low-grade form, and usually causes permanent tissue damage. Macrophage and T-cell recruitment and functions, such as cytokine production, directly contribute to inflammatory pathogenesis. There are many types of diseases and disorders associated with inflammatory responses, all of which are intended to be included under specific embodiments of the present invention.

An "organism" can be single or multi-cellular. The term includes mammals, and, most preferably, humans. Preferred organisms include mice, as the ability to treat or diagnose mice is often predictive of the ability to function in other organisms such as humans.

By "disease or disorder" is meant a state in an organism (e.g., a human) which is recognized as abnormal by members of the medical community. The disease or disorder is characterized by an abnormality in one or more signal transduction pathways in a cell, where the components of the signal transduction pathway include a PYK2 polypeptide and a natural binding partner.

By "abnormality" is meant a level which is statistically different from the level observed in organisms not suffering from such a disease or disorder and may be characterized as either an excess amount, intensity or duration of signal or a deficient amount, intensity or duration of signal. The abnormality in signal transduction may be realized as an abnormality in cell function, viability or differentiation state. Such abnormality in a pathway can be alleviated by action at the PYK2:natural binding partner interaction site in the pathway. An abnormal interaction level may also either be greater or less than the normal level and may impair the normal performance or function of the organism. Thus, it is also possible to screen for agents that will be useful for treating a disease or condition, characterized by an abnormality in the signal transduction pathway, by testing compounds for their ability to affect the interaction between a PYK2 polypeptide and a natural binding partner, since the complex formed by such interaction is part of the signal transduction pathway. However, in some embodiments, the disease or condition may be characterized by an abnormality in the signal transduction pathway even if the level of interaction between the PYK2 polypeptide and natural binding is normal. Further in some embodiments, the defect may result from an inability of a natural binding partner to perform a function on PYK2, or PYK2 to perform a function on a natural binding partner, or both. Finally, in some preferred embodiments, the abnormality does not fall within what is traditionally meant by the signal transduction pathway, e.g. it may involve an activity of PYK2 that does not directly relate to signal transduction.

The term "signal transduction pathway" refers to the molecules that propagate an extracellular signal through the cell membrane to become an intracellular signal. This signal can then stimulate a cellular response. The polypeptide molecules involved in signal transduction processes are typically (but not limited to) receptor and non-receptor protein tyrosine kinases, receptor and non-receptor protein phosphatases, SRC homology 2 and 3 domains, PDZ domain containing proteins, phosphotyrosine binding proteins (SRC homology 2 (SH2) and phosphotyrosine binding (PTB and PH) domain containing proteins), PTB (phosphotyrosine binding) domain that binds to phosphotyrosine as well as non-phosphorylated peptides, PH (pleckstrin homology) domain that binds to phosphoinositides, proline-rich binding proteins (SH3 domain containing proteins), nucleotide exchange factors, and transcription factors.

By "interact" is meant any physical association between polypeptides, whether covalent or non-covalent. This linkage can include many chemical mechanisms, for instance covalent binding, affinity binding, intercalation, coordinate binding and complexation. Examples of non-covalent bonds include electrostatic bonds, hydrogen bonds, and Van der Waals bonds. Furthermore, the interactions between polypeptides may either be direct or indirect. Thus, the association between two given polypeptides may be achieved with an intermediary agent, or several such agents, that connects the two proteins of interest (e.g., a PYK2 polypeptide and a natural binding partner). Another example of an indirect interaction is the independent production, stimulation, or inhibition of both a PYK2 polypeptide and natural binding partner by a regulatory agent. Depending upon the type of interaction present, various methods may be used to measure the level of interaction. For example, the strengths of covalent bonds are often measured in terms of the energy required to break a certain number of bonds (i.e., kcal/mol). Non-covalent interactions are often described as above, and also in terms of the distance between the interacting molecules. Indirect interactions may be described in a number of ways, including the number of intermediary agents involved, or the degree of control exercised over the PYK2 polypeptide relative to the control exercised over the natural binding partner.

By "a PYK2 polypeptide" is meant the PYK2 polypeptide described in U.S. Patent No. 5,837,815 to Lev et al.and WO publication WO 96/18738 by Lev et al., both hereby incorporated by reference herein in their entirety including tables, figures, and drawings. The isolation and characterization of the PYK2 polypeptide is also fully described therein. The PYK2 polypeptide can be encoded by a full-length nucleic acid sequence or any portion of the full-length nucleic acid sequence, so long as a functional activity of the polypeptide is retained. Preferred functional activities include, but are not limited to, the ability to phosphorylate and regulate RAK and/or other potassium channels. A variety of methodologies known in the art can be utilized to obtain PYK2 polypeptides for use in the methods of the invention.

The term "natural binding partner" refers to polypeptides, lipids, small molecules, or nucleic acids that bind to kinases in cells. A change in the interaction between a kinase and a natural binding partner can manifest itself as an increased or decreased probability that the interaction forms, or an increased or decreased concentration of kinase/natural binding partner complex. Binding is understood to include interactions such as phosphorylation or dephosphorylation, for example.

The term "modulates" refers to the ability of a compound to alter the interaction of PYK2 and a natural binding partner. The Km of a compound is preferably between 100 µM and 1 µM, more preferably between 1 µM and 100 nM, most preferably between 100 nM and 1 nM. A modulator preferably promotes or disrupts the interaction of PYK2 and a natural binding partner. Alternatively, the modulator may increase or decrease the cellular activity of the kinase, including phosphorylation. Kinase activity is preferably the phosphorylation of a natural binding partner on tyrosine, serine, or threonine residues. Changes in the interaction with a natural binding partner can also include increasing or decreasing the probability that a complex forms between the kinase and a natural binding partner. A modulator preferably increases the probability that such a complex forms between the kinase and the natural binding partner, and most preferably decreases the probability that a complex forms between the kinase and the natural binding partner. In some preferred embodiments the interaction includes actions of the natural binding partner on PYK2.

The term "complex" refers to an assembly of at least two molecules bound to one another. Signal transduction complexes often contain at least two protein molecules bound to one another. For instance, a protein tyrosine kinase receptor, GRB2, SOS, RAF, and RAS assemble to form a signal transduction complex in response to a mitogenic ligand.

By "disrupt" is meant that the interaction between the PYK2 polypeptide and a natural binding partner is reduced either by preventing expression of the PYK2 polypeptide, or by preventing expression of the natural binding partner, or by specifically preventing interaction of the naturally synthesized proteins or by interfering with the interaction of the proteins.

By "promote" is meant that the interaction between a PYK2 polypeptide and a natural binding partner is increased either by increasing expression of a PYK2 polypeptide, or by increasing expression of a natural binding partner, or by decreasing the dephosphorylating activity of the corresponding regulatory TP (or other phosphatase acting on other phosphorylated signaling components), by promoting interaction of the PYK2 polypeptide and natural binding partner or by prolonging the duration of the interaction.

The term "activates" refers to increasing the cellular activity of the kinase. The term "inhibit" refers to decreasing the cellular activity of the kinase. Kinase activity is preferably the phosphorylation of a natural binding partner on tyrosine, threonine, or serine residues. Changes in the interaction with a natural binding partner can also include increasing or decreasing the probability that a complex forms between the kinase and a natural binding partner. A modulator preferably increases the probability that such a complex forms between the kinase and the natural binding partner, and most preferably decreases the probability that a complex forms between the kinase and the natural binding partner.

In preferred embodiments of methods for screening for compounds potentially useful for treating or preventing inflammatory response-related diseases or disorders involving the interaction of PYK2 and a natural binding partner, the inflammatory response-related disease or disorder is selected from the group consisting of inflammatory bowel diseases and connective tissue diseases. Preferably, the inflammatory bowel diseases are selected from the group consisting of ulcerative colitis and Crohn's Disease and the connective tissue diseases are selected from the group consisting of rheumatoid arthritis, systemic lupus erythematosus, progressive systemic sclerosis, mixed connective tissue disease, and Sjogren's syndrome.

Macrophage function and the production of cytokines by macrophages and other cells associated with the inflammatory response directly contribute to the pathophysiologic progression of the diseases. The importance of PYK2 in these disease processes is indicated by the decreased production of cytokines in cells from *pyk2* -/- mice and in macrophage cell lines expressing kinase inactive PYK2. Thus, inhibiting PYK2 function in inflammatory cells should alleviate some of the pathologic consequences associated with these diseases.

The term "inflammatory bowel disease" as used herein, refers to inflammatory diseases of the bowel, many of which are of unknown etiology, including Crohn's disease and ulcerative colitis.

The term "Crohn's Disease" as used herein, refers to a chronic granulomatous inflammatory disease of unknown etiology, involving any part of the gastrointestinal tract from mouth to anus, but commonly involving the terminal ileum and/or colon with scarring and thickening of the bowel wall. It frequently leads to intestinal obstruction and fistula and abscess formation and has a high rate of recurrence after treatment.

By "ulcerative colitis" is meant chronic, recurrent ulceration in the colon, chiefly of the mucosa and submucosa, of unknown cause. The rectum is almost always involved. It is manifested clinically by cramping abdominal pain, rectal bleeding, and loose discharges of blood, pus, and mucus with scanty fecal particles. Complications include hemorroids, abscesses, fistulas, perforation of the colon, pseudopolyps, and carcinoma.

The term "connective tissue diseases" as used herein refers to heterogeneous disorders which share certain common features, including inflammation of skin, joints, and other structures rich in connective tissue, as well as altered patterns of immunoregulation, including production of autoantibodies and abnormalities of cell-mediated immunity. While certain distinct clinical entities may be defined, manifestations may vary considerably from one patient to the next and overlap of clinical features between and among specific diseases is common.

The term "rheumatoid arthritis" as used herein refers to a chronic systemic disease primarily of the joints, usually polyarticular, marked by inflammatory changes in the synovial membranes and articular structures and by muscle atrophy and rarefaction of the bones. Persistent inflammatory synovitis usually involves the peripheral joints in a symmetrical fashion, marked by cartilaginous destruction, bony erosions, and joint deformation. Infiltration of inflammatory cells is common. Forms of rheumatoid arthritis include, but are not limited to, juvenile, chronic villous, cricoarytenoid, deformans, degenerative, mutilans, and proliferative.

The term "systemic lupus erythematosus" as used herein, refers to a disease in which tissues and cells are damaged by deposition of pathogenic antibodies and immune complexes. B-cell hyperactivity, production of autoantibodies with specificity for nuclear antigenic determinants, and abnormalities of T-cell function occur. It may involve virtually any organ system and follows a course of exacerabation followed by remission. A common feature is the ,alar "butterfly" rash.

The term "progressive systemic sclerosis" as used herein refers to a multisystem disorder characterized by inflammatory, vascular, and fibrotic changes of skin and various internal organ sytems (chiefly GI tract, lungs, heart, and kidney). Primary event may be endothelial cell injury with eventual intimal proliferation, fibrosis, and vessel obliteration. Clinical manifestations include, but are not limited to, Raynaud's phenomenon, scleroderma (fibrosis of the skin), hypertension, and renal failure.

The term "mixed connective tissue disease" as used herein, refers to syndrome characterized by a combination of clinical features similar to those of systemic lupus erythematosus, progressive systemic sclerosis, polymyositis, and rheumatoid arthritis. Unusually high titers of circulating antibodies to a nuclear ribonucleoprotein are found. Clinical manifestations include Raynaud's phenomenon, polyarthritis and pulmonary fibrosis among others.

The term "Sjogren's syndrome" as used herein refers to an immunologic disorder characterized by progressive destruction of exocrine glands leading to mucosal and conjunctival dryness (sicca syndrome). Affected tissues show lymphocyte infiltration and immune-complex deposition.

In preferred embodiments of methods of identifying compounds, the one or more compounds modulate (inhibit or promote) the interaction of PYK2 and a natural binding partner in vitro. An example of an in vitro method involves growing cells (i.e., in a dish) that either naturally or recombinantly express a G-coupled protein receptor, PYK2, and RAK. The test compound preferably is added at a concentration from 0.1 µM to 100 µM and the mixture preferably is incubated from 5 minutes to 2 hours. The ligand is added to the G-coupled protein receptor preferably for 5 to 30 minutes and the cells are lysed. RAK is isolated using immunoprecipitation or ELISA by binding to a specific monoclonal antibody. The amount of phosphorylation compared to cells that were not exposed to a test compound is measured using an anti-phosphotyrosine antibody (preferably polyclonal). Alternatively, in other methods of identifying compounds, the one or more compounds modulate (inhibit or promote) PYK2 and natural binding partner interactions *in vivo*.

In other methods of identifying compounds, the interaction is selected from the group consisting of PYK2 phosphorylation, PYK2 natural binding partner phosphorylation, PYK2 de-phosphorylation, PYK2 natural binding partner de-phosphorylation, and complex formation between PYK2 and a natural binding partner.

Examples of compounds that could be tested in such screening methods include tyrphostins, quinazolines, quinoxolines, quinolines, and indolinones. Publications describing representative examples of these compounds and methods of making are given in the Detailed Description of the Invention.

A second aspect of the invention features a method for diagnosis of a disease or disorder, wherein said disease or disorder is characterized by an inflammatory response involving an abnormality in a signal transduction pathway that includes an interaction between a PYK2 polypeptide and a natural binding partner, comprising detecting the level of said interaction as an indication of said disease or disorder.

By "diagnosis" is meant any method of identifying a symptom normally associated with a given disease or condition. Thus, an initial diagnosis may be conclusively established as correct by the use of additional confirmatory evidence such as the presence of other symptoms. Current classification of various diseases and conditions is constantly changing as more is learned about the mechanisms causing the diseases or conditions. Thus, the detection of an important symptom, such as the detection of an abnormal level of interaction between PYK2 polypeptides and natural binding partners may form the basis to define and diagnose a newly named disease or condition. For example, conventional cancers are classified according to the presence of a particular set of symptoms. However, a subset of these symptoms may both be associated with an abnormality in a particular signaling pathway, such as the ras²¹ pathway and in the future these diseases may be reclassified as ras²¹ pathway diseases regardless of the particular symptoms observed.

In preferred embodiments of methods for screening for diagnosis of inflammatory response-related diseases or disorders involving the interaction of PYK2 and a natural binding partner, the inflammatory response-related disease or disorder is selected from the group consisting of inflammatory bowel diseases and connective tissue diseases. Preferably, the inflammatory bowel diseases are selected from the group consisting of ulcerative colitis and Crohn's Disease and the connective tissue diseases are selected from the group consisting of rheumatoid arthritis, systemic lupus erythematosus, progressive systemic sclerosis, mixed connective tissue disease, and Sjogren's syndrome.

A third aspect of the invention features a method for treating or preventing a disease or disorder, wherein said disease or disorder is characterized by an inflammatory response involving an abnormality in a signal transduction pathway that includes an interaction between a PYK2 polypeptide and a natural binding partner, comprising administering to a patient in need of such treatment one or more compounds preferably in a pharmaceutically acceptable composition, wherein said one or more compounds modulate said interaction.

In preferred embodiments of methods for treating or preventing inflammatory response-related diseases or disorders involving the interaction of PYK2 and a natural binding partner, the inflammatory response-related disease or disorder is selected from the group consisting of inflammatory bowel diseases and connective tissue diseases. Preferably, the inflammatory bowel diseases are selected from the group consisting of ulcerative colitis and Crohn's Disease and the connective tissue diseases are selected from the group consisting of rheumatoid arthritis, systemic lupus erythematosus, progressive systemic sclerosis, mixed connective tissue disease, and Sjogren's syndrome.

In other preferred embodiments of the methods for treating or preventing inflammatory response-related diseases or disorders involving the interaction of PYK2 and a natural binding partner, the one or more compounds modulate (inhibit or promote) the interaction in vitro and/or *in vivo.* In some preferred embodiments, the interaction is selected from the group consisting of PYK2 phosphorylation, PYK2 natural binding partner phosphorylation, PYK2 de-phosphorylation, PYK2 natural binding partner de-phosphorylation, and complex formation between PYK2 and a natural binding partner.

In yet other preferred embodiments of methods for treating or preventing inflammation-related diseases or disorders involving the interaction of PYK2 and a natural binding partner, the one or more compounds is selected from the group consisting of tyrphostins, quinazolines, quinoxolines, quinolines, and indolinones.

In preferred embodiments the agent is therapeutically effective and preferably has an EC₅₀, or IC₅₀ of less than or equal to 100 µM, even more preferably less than or equal to 50 µM, and most preferably less than or equal to 10 µM. Such lower EC₅₀' S or IC₅₀'s are advantageous since they allow lower concentrations of molecules to be used *in vivo* or *in vitro* for therapy or diagnosis. The discovery of molecules with such low EC₅₀'s and IC₅₀'S enables the design and synthesis of additional molecules having similar potency and effectiveness. Generally, a therapeutically effective amount is between about 1 nmol and 1 µmol of the molecule, depending on its EC₅₀ or IC₅₀, and on the age and size of the patient, and the disease associated with the patient.

The term "preventing" refers to decreasing the probability that an organism contracts or develops an abnormal condition. Thus, in these cases a patient would include someone who is thought to be at risk for contracting an abnormal condition. Persons skilled in the art would be able to identify persons who would be considered at risk from contracting an abnormal condition.

The term "treating" refers to having a therapeutic effect and at least partially alleviating or abrogating an abnormal condition in the organism. In this case the patient is already been identified as having an abnormal condition.

The term "therapeutic effect" refers to the inhibition or activation of factors causing or contributing to the abnormal condition. A therapeutic effect relieves to some extent one or more of the symptoms of the abnormal condition. In reference to the treatment of abnormal conditions, a therapeutic effect can refer to one or more of the following: (a) an increase or decrease in the infiltration of cells; (b) inhibition of (i.e., slowing or stopping) or increase in cell movement; (c) relieving to some extent one or more of the symptoms associated with the abnormal condition; and (d) enhancing or inhibiting the function of the affected population of cells. Compounds demonstrating efficacy against abnormal conditions can be identified as described herein.

The term "abnormal condition" refers to a function in the cells or tissues of an organism that deviates from their normal functions in that organism. An abnormal condition can relate to cell proliferation, cell differentiation, cell function, or cell survival.

Abnormal cell infiltration conditions include, but are not limited to, rheumatoid arthritis and chronic inflammatory bowel disease.

The term "aberration", in conjunction with the function of a kinase in a signal transduction process, refers to a kinase that is over- or under-expressed in an organism, mutated such that its catalytic activity is lower or higher than wild-type protein kinase activity, mutated such that it can no longer interact with a natural binding partner, is no longer modified by another protein kinase or protein phosphatase, or no longer interacts with a natural binding partner.

The term "administering" relates to a method of incorporating a compound into cells or tissues of an organism. The abnormal condition can be prevented or treated when the cells or tissues of the organism exist within the organism or outside of the organism. Cells existing outside the organism can be maintained or grown in cell culture dishes. For cells harbored within the organism, many techniques exist in the art to administer compounds, including (but not limited to) oral, parenteral (e.g. intra-venous, intra-muscular, sub-cutaneous, and intra-articular) and aerosol applications. The compounds may also be administered in a depot or sustained release formulation. For cells outside of the organism, multiple techniques exist in the art to administer the compounds, including (but not limited to) cell microinjection techniques, simple diffusion, and carrier techniques.

The term "pharmaceutically acceptable" or "pharmaceutical" as used herein refers to solutions or components of the pharmaceutical composition that do not prevent the therapeutic compound from exerting a therapeutic effect and do not cause unacceptable adverse side effects. Examples of pharmaceutically acceptable reagents are provided in *The United States Pharmacopeia The National Formulary*, United States Pharmacopeial Convention, Inc., Rockville, MD 1990 and *FDA Inactive* Ingredient Guide 1990, 1996 issued by the Division of Drug Information Resources (both are hereby incorporated by reference herein, including any drawings). Unacceptable side effects vary for different diseases. Generally, the more severe the disease the more toxic effects which will be tolerated. Unacceptable side effects for different diseases are known in the art.

The term "physiologically acceptable" defines a carrier or diluent that does not cause significant irritation to an organism and preferably does not abrogate the biological activity and properties of the compound.

The term "carrier" defines a chemical compound that facilitates the incorporation of a compound into cells or tissues. For example dimethyl sulfoxide (DMSO) is a commonly utilized carrier as it facilitates the uptake of many organic compounds into the cells or tissues of an organism.

The term "diluent" defines chemical compounds diluted in water (or another solvent) that will dissolve the compound of interest as well as stabilize the biologically active form of the compound. Many salts dissolved in buffered solutions are utilized as diluents in the art. One commonly used buffered solution is phosphate buffered saline because it mimics the salt conditions of human blood. Because buffer salts can control the pH of a solution at low concentrations, a diluent rarely modifies the biological activity of a compound.

The term "solvent" as used herein refers to a chemical compound that facilitates the solubilization of compounds of the invention. Examples of solvents include, but are not limited to, pharmaceutically acceptable alcohols, such as ethanol and benzyl alcohol; polyoxyhydrocarbyl compounds, such as poly(ethylene glycol); pharmaceutically acceptable surfactants such as CREMOPHOR® EL; polyglycolized lipids, such as GELUCIRE® and LABRASOL®: and pharmaceutically acceptable oils, such as miglyol 812.

The term "pharmaceutically acceptable alcohol" as used herein refers to alcohols that are liquids at about room temperature (approximately 20 °C). These include propylene glycol, ethanol, 2-(2-ethoxyethoxy)ethanol (TRANSCUTOL®, Gattefosse, Westwood, NJ 07675), benzyl alcohol, and glycerol.

The term "polyoxyhydrocarbyl compound" as used herein refers to a water soluble carbohydrate such as glucose, sucrose, maltotriose, and the like; water soluble carbohydrate derivatives such as gluconic acid and mannitol, and oligosaccharides; and water soluble polymers such as polyvinylpyrrolidone, poly(vinyl alcohol), and in particular, polyethers such as other polyoxyalkylenes including poly(ethylene glycol) or other water soluble mixed oxyalkylene polymers and the polymeric form of ethylene glycol. Although polyoxyhydrocarbyl compounds preferably-contain more than one carbon, oxygen, and hydrogen atom, some molecules such as poly(ethylene imine) are also included.

A particularly preferred class of solubilizing polyoxyhydrocarbyl moieties comprises poly(ethylene glycol) (PEG) and PEG derivatives, such as PEG monomethyl ether. Other suitable PEG derivatives include PEG-silicon derived ethers. Many of these polymers are commercially available in a variety of molecular weights. Others may be conveniently prepared from commercially available materials, such as by coupling of amino-PEG moiety to a haloalkyl silyl or silane moiety.

Suitable PEGs may vary in molecular weight from about 200 g/mol to about 20,000 g/mol or more, more preferably 200 g/mol to 5,000 g/mol, even more preferably 250 g/mol to 1,000 g/mol, and most preferably 250 g/mol to 500 g/mol. The choice of a particular molecular weight may depend on the particular compound chosen and its molecular weight and degree of hydrophobicity, as well as the particular application for which the formulation is to be used.

The term "pharmaceutically acceptable surfactant" as used herein refers to a compound that can solubilize compounds of the invention into aqueous solutions, if necessary. Preferably for parenteral formulations, the surfactant is a non-ionic surfactant. Examples of pharmaceutically acceptable surfactants include POLYSORBATE 80 and other polyoxyethylene sorbitan fatty acid esters, glyceryl monooleate, polyvinyl alcohol, ethylene oxide copolymers such as PLURONIC® (a polyether) and TETRONIC® (BASF), polyol moieties, and sorbitan esters. Preferably ethoxylated castor oils, such as CREMOPHOR® EL, are used for the formulation of some compounds.

The term "ethoxylated castor oil" as used herein refers to castor oil that is modified with at least one oxygen containing moiety. In particular the term refers to castor oil comprising at least one ethoxyl moiety.

Further, the term "pharmaceutically acceptable surfactant" as used herein in reference to oral formulations, includes pharmaceutically acceptable non-ionic surfactants (for example polyoxyethylene-polypropylene glycol, such as POLOXAMER® 68 (BASF Corp.) or a mono fatty acid ester of polyoxyethylene (20) sorbitan monooleate (TWEEN® 80), polyoxyethylene (20) sorbitan monostearate (TWEEN® 60), polyoxyethylene (20) sorbitan monopalmitate (TWEEN® 40), polyoxyethylene (20) sorbitan monolaurate (TWEEN® 20) and the like); polyoxyethylene castor oil derivatives (for example, polyoxyethyleneglycerol-ttiricinoleate or polyoxyl 35 castor oil (CREMOPHOR® EL, BASF Corp.), polyoxyethyleneglycerol oxystearate (CREMOPHOR® RH 40 (polyethyleneglycol 40 hydrogenated castor oil) or CREMOPHOR® RH 60 (polyethyleneglycol 60 hydrogenated castor oil), BASF Corp.) and the like); or a pharmaceutically acceptable anionic surfactant.

The term "polyglycolized lipids" as used herein refers to mixtures of monoglycerides, diglycerides, or triglycerides and polyethyleneglycol monoesters and diesters formed by the partial alcoholysis of vegetable oil using PEG of 200 g/mol to 2,000 g/mol or by the esterification of fatty acids using PEG 200 g/mol to 2,000 g/mol and glycerols. Preferably these include GELUCIRE® 35/10, GELUCIRE® 44/14, GELUCIRE® 46/07, GELUCIRE® 50/13, GELUCIRE® 53/10, and LABRASOL®.

The term "pharmaceutically acceptable oils" as used herein refers to oils such as mineral oil or vegetable oil (including safflower oil, peanut oil, and olive oil), fractionated coconut oil, propylene glycol monolaurate, mixed triglycerides with caprylic acid and capric acid, and the like. Preferred embodiments of the invention feature mineral oil, vegetable oil, fractionated coconut oil, mixed triglycerides with caprylic acid, and capric acid. A highly preferred embodiment of the invention features Miglyol® 812 (available from Huls America, USA).

In preferred embodiments, the methods described herein involve identifying a patient in need of treatment. Those skilled in the art will recognize that various techniques may be used to identify such patients.

A fourth aspect of the invention features a composition comprising one or more compounds identified by any of the methods of the invention described above or herein. Preferably, this composition is useful for treating or preventing a disease or disorder, where the disease or disorder is characterized by an inflammatory response involving an abnormality in a signal transduction pathway that includes an interaction between a PYK2 polypeptide and a natural binding partner.

In preferred embodiments, the inflammatory response-related disease or disorder is selected from the group consisting of inflammatory bowel diseases and connective tissue diseases. Preferably, the inflammatory bowel diseases are selected from the group consisting of ulcerative colitis and Crohn's Disease and the connective tissue diseases are selected from the group consisting of rheumatoid arthritis, systemic lupus erythematosus, progressive systemic sclerosis, mixed connective tissue disease, and Sjogren's syndrome.

A fifth aspect of the invention features methods of making compounds potentially useful to treat or to prevent a disease or disorder, where the disease or disorder is characterized by an inflammatory response that is characterized by an abnormality in a signal transduction pathway, and where the signal transduction pathway includes an interaction between a PYK2 polypeptide and a natural binding partner, comprising assaying one or more potential compounds for those able to modulate the interaction as an indication of a useful compound and synthesizing the identified compounds. References describing methods of synthesizing the identified compounds are indicated in the Detailed Description of the Invention.

In preferred embodiments, the inflammatory response-related disease or disorder is selected from the group consisting of inflammatory bowel diseases and connective tissue diseases. Preferably, the inflammatory bowel diseases are selected from the group consisting of ulcerative colitis and Crohn's Disease and the connective tissue diseases are selected from the group consisting of rheumatoid arthritis, systemic lupus erythematosus, progressive systemic sclerosis, mixed connective tissue disease, and Sjogren's syndrome.

A sixth aspect of the invention features kits comprising a composition comprising one or more compounds identified by any of the methods of the invention described above or herein. Preferably, this composition is useful for treating or preventing a disease or disorder, where the disease or disorder is characterized by an inflammatory response involving an abnormality in a signal transduction pathway that includes an interaction between a PYK2 polypeptide and a natural binding partner. The kit preferably further comprises instructions for use either on a label or using other suitable means as discussed herein in the Detailed Description of the Invention.

In other preferred embodiments, the kit comprises additional container means containing one or more of the following: diluents, carriers, and solvents. Such containers include small glass containers, plastic containers, or strips of plastic or paper. Such containers allow the efficient transfer of contents from one container to another, such that the contents are not contaminated and the contents can be added in a quantitative fashion from one compartment to another. The kit may additionally comprise means for administering the composition. One skilled in the art will readily recognize that the compositions of the instant invention can be readily incorporated into one of the established kit formats that are well-known in the art.

In preferred embodiments, the composition contained in the kit is useful for treating or preventing an inflammatory response-related disease or disorder selected from the group consisting of inflammatory bowel diseases and connective tissue diseases. Preferably, the inflammatory bowel diseases are selected from the group consisting of ulcerative colitis and Crohn's Disease and the connective tissue diseases are selected from the group consisting of rheumatoid arthritis, systemic lupus erythematosus, progressive systemic sclerosis, mixed connective tissue disease, and Sjogren's syndrome.

Other features and advantages of the invention will be apparent from the following description of the preferred embodiments thereof, and from the claims.

### BRIEF DESCRIPTION OF THE FIGURES

These figures are provided for illustration only, and are not considered necessary to disclose the invention.

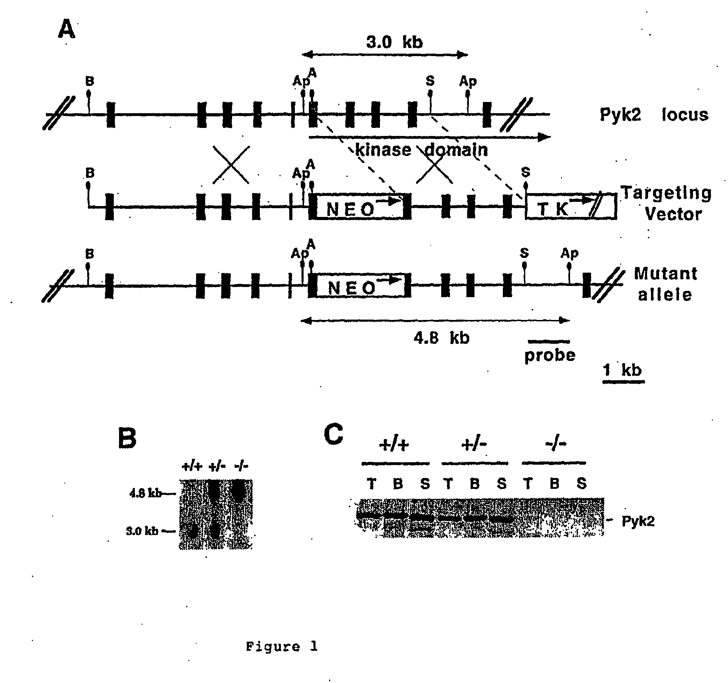

Figures 1a, 1b, and 1c show the targeted disruption of the *pyk2* gene in mice. Partial restriction maps of murine *pyk2* locus, the regions used as a targeting vector, and the expected mutant allele are shown in Fig 1a. Solid boxes indicate exons. The probe was used for Southern blot hybridization analysis of the genomic DNA from the ES cells and tail biopsy analysis. The solid line under the *pyk2* locus marks the PYK2 kinase domain. Arrows mark the expected *Apa1* fragments of the wild-type and mutant alleles. Fig. 1b shows Southern blot hybridization analysis of the mouse tail DNA digested with *Apa1.* Fig. 1c shows immunoblot analysis with anti-PYK2 antibodies of lysates from Thymus(T), Brain (B), and Spleen (S) from wild-type, *pyk2* +/-, and *pyk2* -/- mice.

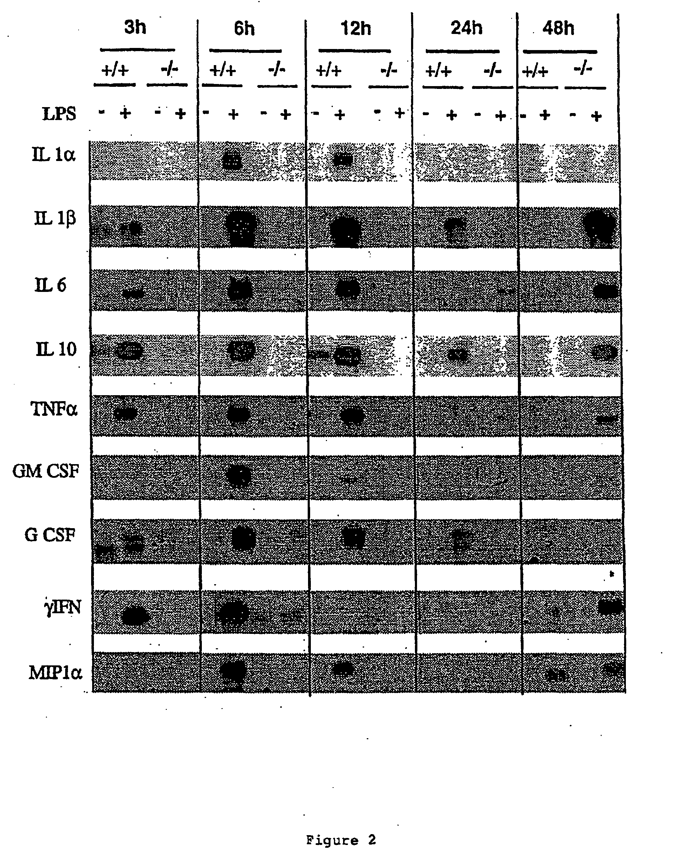

Figure 2 shows RT-PCR (reverse transcriptase-polymerase chain reaction) analysis of cytokine productions from wild-type +/+ and *pyk2 -*/thioglycollated-elicited peritoneal macrophages stimulated by LPS. Thioglycollate-elicited peritoneal macrophages were incubated with or without LPS (10 µg/mL) for 3h, 6h, 12h, 24h, and 48h. Total RNAs were isolated from cells at each time point and after normalization of the amount of mRNA in the total RNAs by RT-PCR of actin mRNA, total RNAs were subjected to RT-PCR with specific probes for various cytokines.

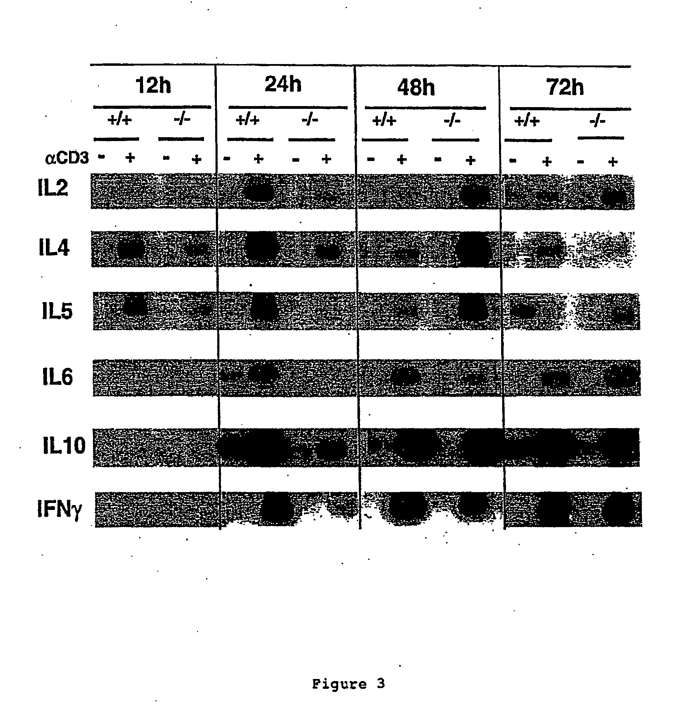

Figure 3 shows RT-PCR analysis of the cytokine productions from wild-type +/+ and *pyk2* -/- splenocytes stimulated by anti-murine CD3 antibodies. Splenocytes were incubated with or without anti-CD3 (1 µg/mL) for 12h, 24h, 48h and 72h. Total RNAs were isolated from cells at each time point and after normalization of the amount of mRNA in the total RNAs by RT-PCR of the actin mRNA, total RNAs were subjected to RT-PCR with specific probes for various cytokines.

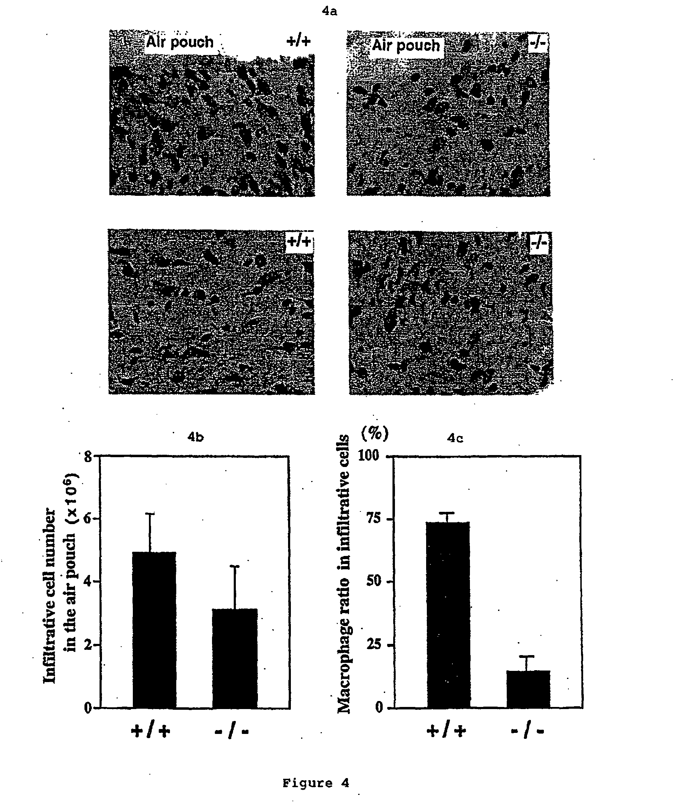

Figures 4a, 4b, and 4c demonstrate Carageenen induced cellular infiltration in murine air pouches. Fig. 4a shows tissues from wild type +/+ and *pyk2.*-/- air pouches that were treated with Carageenen for 10 hours after injection. Samples were formalin-fixed and paraffin-embedded. Sections were stained with Hematoxylin and Eosin. Figure 4b shows a number of cells infiltrating into wild type +/+ and *pyk2* -/- air pouches 10 hours after injection with Carageenen. Figure 4c shows a fraction of cells infiltrating into wild-type +/+ and *pyk2* -/- mice air pouches 10 hours after injection with Carageenen.

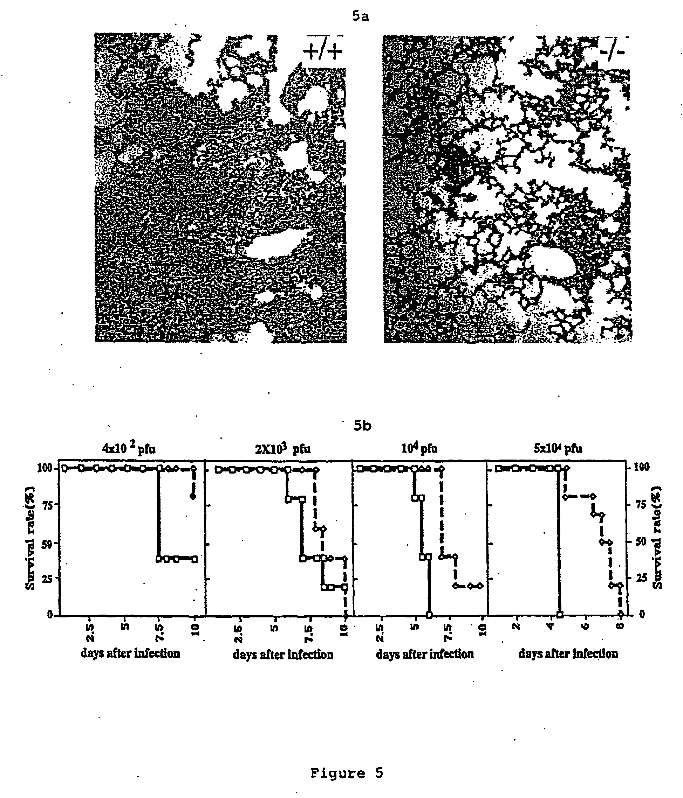

Figures 5a and 5b demonstrate influenza virus-induced inflammation. Fig. 5a shows histologic sections that were made of the lung from influenza virus-infected wild-type +/+ and pyk2-/- mice 4 days after infection with the virus. Lungs were formalin-fixed and paraffin-embedded. Sections were stained with Hematoxylin and Eosin. Fig. 5b shows the time course of the survival of mice at different doses of influenza virus.

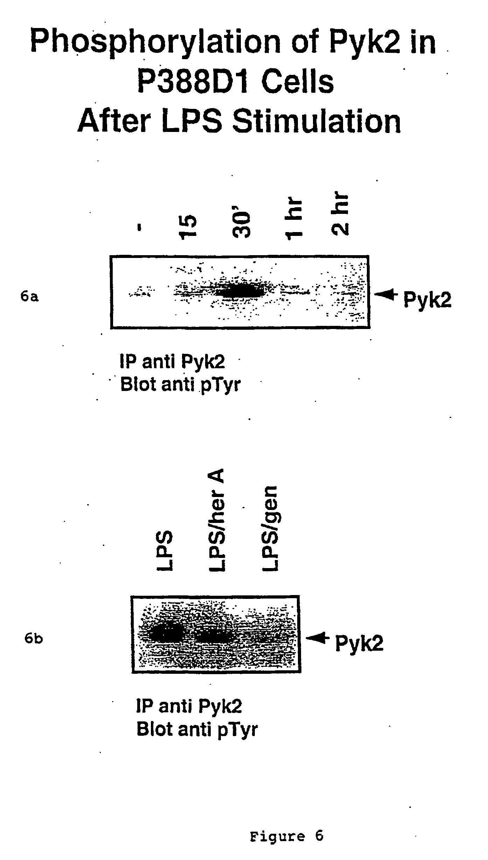

Figures 6a and 6b show the tyrosine phosphorylation of PYK2 in macrophage cell lines in response to LPS (lipopolysaccharide) and tyrosine kinase inhibitors. Cells were either starved overnight in 0.5% serum and stimulated with LPS for variable time intervals (Figure 6a), or were pre-treated with either herbimycin A or genestein for 4 hours and then stimulated with LPS for 30 minutes (Figure 6b). After stimulation, cells were washed, lysed, and immunoprecipitated with anti-PYK2 antibody. Immunnoprecipitates (IP) were then analyzed by western blotting.

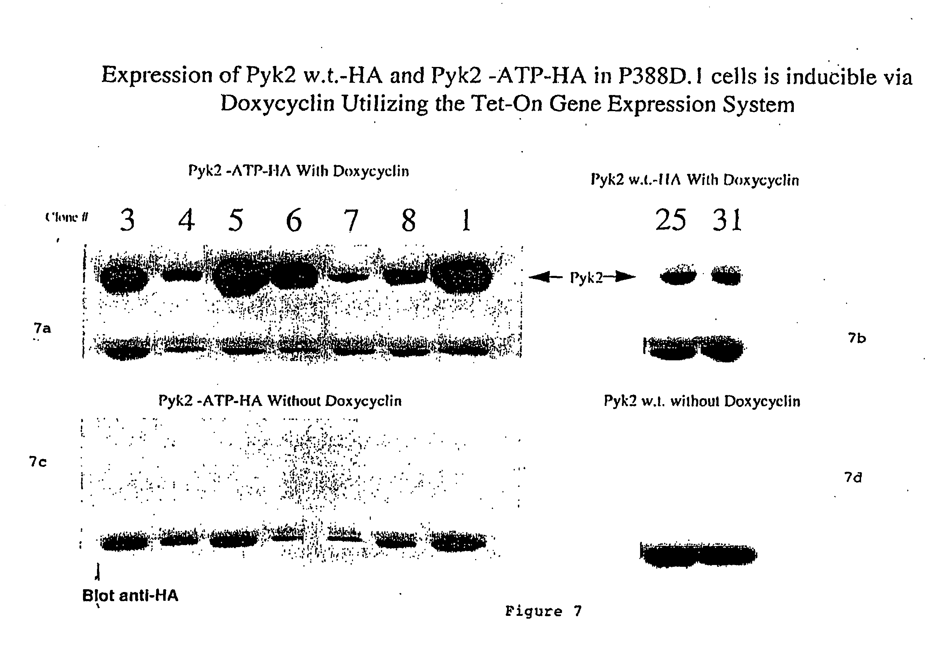

Figure 7a, 7b, 1c, and 7d demonstrate that expression of both the PYK2 wild-type and the dominant negative kinase mutant are inducible in P388D1 cells. The murine macrophage cell line (P388D1) was serially transfected with doxycycline (DOX) inducible plasmids. In the first transfection, a regulator plasmid was introduced, and stable, drug-resistant clones were established. Two clones were then selected and transfected with doxycycline-responsive plasmids containing either HA-tagged (hemagglutinin), wild-type PYK2 or the kinase dead, dominant-negative PYK2 mutant (also HA-tagged). After drug selection, stable clones containing the wild-type (Figures 7b and 7d) or the dominant-negative mutant (Figures 7a and 7c) were screened for expression of the appropriate protein in the presence (Figures 7 a and 7b) and absence (Figures 7c and 7d) of doxycyclin by western blotting.

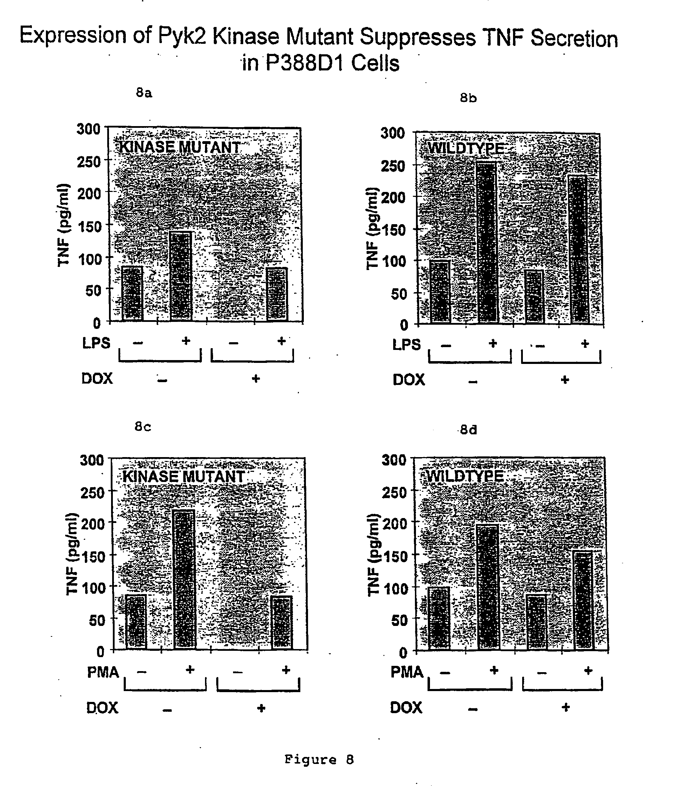

Figures 8a, 8b, 8c, and 8d compare the secretion of TNF-α by wild-type and kinase mutant P388D1 cells in response to activators of macrophage function or PYK2 activity. Figures 8a and 8b show the effect of LPS in the presence and absence of DOX on TNF-α secretion from kinase mutant (dominant negative; Fig. 8a) and wild-type (Fig. 8b) cells. Figures 8c and 8d show the effect of PMA in the presence and absence of DOX on TNF-α secretion from kinase mutant (Fig. 8c) and wild-type (Fig. 8d) cells. Induction of P388D1 cells in the presence of doxicycline was carried out for 48 hours followed by stimulation with LPS (1 µg/mL) or PMA (1 µg/mL) for 18 hrs. Secreted TNF-α was measured by ELISA.

### DETAILED DESCRIPTION OF THE INVENTION

The current invention demonstrates for the first time in an *in vivo* mouse model and a cellular model the link between PYK2 and the inflammatory response. To demonstrate the role of PYK2 *in vivo,* knockout mice lacking the *pyk2* gene were created using molecular genetic techniques. The inflammatory response of the knockout mice was compared with the corresponding mice not containing a *pyk2* deletion using two *in vivo* models: an influenza virus model and a subcutaneous carageenen air pouch model.

In the influenza model, infection with the influenza virus leads to an overwhelming pulmonary inflammatory response and eventually death. It was found that the *pyk2* knockout mice survived significantly longer than control mice expressing the *pyk2* gene (48 hours). Histologic examination of the lungs of the knockout and control mice showed that in the lungs of the *pyk2* knockout mice there was a significantly lower infiltration of inflammatory cells including PMNs (polymorphonuclear leukocytes), macrophages, and T-cells. *In vitro* experiments showed that macrophages and splenocytes from *pyk2* -/- mice produced decreased amounts of cytokines and chemokines, including IL-1α, IL-1β, IL-6, IL-10, TNF-α, GMCSF, IFN-γ, and MIP1-α. Lower production of cytokines and chemokines such as TNF-α and MIP1-α (among others) results in attenuated recruitment and decreased activation of macrophages, T-cells and other hematopoietic and nonhematopoietic cells involved in the inflammatory response. In addition, macrophages from knockout mice had decreased motility in vitro. The attenuated cytokine response and migration defect in vitro correlate with the enhanced survival and decreased pulmonary cellular infiltrate in *pyk2* -/- mice following influenza challenge.

In the subcutaneous carageenen air pouch model, a subcutaneous air pouch is surgically induced on the hind flank of the mouse. The air pouch is then filled with the immunogen carageenen, a substance that induces an inflammatory response. After 10 hours, the number and type of cells infiltrating the pouch are enumerated. Histologic examination revealed a decrease in the number of cells infiltrating the carageenen filled pouch of *pyk2* -/- mice compared to controls. In addition there was a significant decrease in the number of macrophages present at the site of inflammation.

To evaluate the role of PYK2 in mature macrophage cells that express wild-type PYK2, kinase inactive PYK2 protein was introduced into a normal macrophage cell line. Expression of the kinase inactive PYK2 should function as a "dominant-negative" inhibitor and abrogate the function of the endogenous wild-type PYK2 protein in these macrophages. Expression of the kinase inactive PYK2 protein decreased secretion of TNF-α in response to LPS, a physiologically relevant inducer of the inflammatory response.

These data confirm the role for PYK2 in cytokine release and support the importance of PYK2 function in inflammation. These experiments indicate that treatments that inhibit the functioning of PYK2 will be useful to decrease excessive inflammatory responses, whereas treatments to enhance the functioning of PYK2 will be useful to augment inadequate immune responses.

### I. PYK2 and Signal Transduction

PYK-2 is a non-receptor tyrosine kinase that is activated by binding of ligand to G-coupled protein receptors such as bradykinin, acetylcholine, and CXCR4 or CCR5. PYK2 has a predicted molecular weight of 111 kD and contains five domains: (1) a relatively long N-terminal domain; (2) a kinase catalytic domain; (3) a proline rich domain; (4) another proline rich domain; and (5) a C-terminal domain.

The C-terminal domain of PYK2 has 62% similarity to the C-terminal domain of another non-receptor tyrosine kinase, focal adhesion kinase (FAK), which is also activated by G-coupled proteins. The overall similarity between PYK2 and FAK is 52%. However, the expression of PYK2 does not correspond with the expression of FAK. PYK2 exhibits diffuse cytoplasmic localization unlike the preferential localization of Fak in focal adhesion areas. PYK2 is expressed in a variety of cells including neural tissues, hematopoietic cells, some tumor cell lines, and immune-related cells. PYK2 is highly expressed in the nervous system and in the adult rat brain.

PYK2 enzymatic activity is positively regulated by phosphorylation on tyrosine and results in response to binding of bradykinin, TPA, calcium ionophore, carbachol, TPA + forskolin, and membrane depolarization. Activated PYK2 is known to phosphorylate and thus suppress the activity of the delayed rectifier-type K+ channel, termed RAK (also called Kvi.2, RBK2, RCK5 and NGKI), that is highly expressed in the brain and cardiac atria. In the same system, FAK does not phosphorylate RAK. PYK2 activation may provide a rapid and highly localized control mechanism for ion channel function and kinase activation induced by stimuli that elevate intracellular calcium.

PYK2 is activated by, and phosphorylated on tyrosine residues in response to a variety of extracellular signals that lead to calcium influx or calcium release from internal stores. Calcium influx leads to the activation of PYK2, tyrosine phosphorylation of Shc, recruitment of Grb2/Sos and activation of the MAP kinase signaling pathway that relays signals to the cell nucleus. Overexpression of PYK2 also leads to activation of MAP kinase. Thus, PYK2 may provide a link between G-protein coupled receptors and calcium influx and the MAP kinase signaling pathway; a pathway that relays signals from the cell surface to regulate transcriptional events in the nucleus.

These results reveal a role for PYK2 in activation of the MAP kinase signaling pathway by ion channels, calcium influx and G-protein coupled receptors and provide a mechanism for signal transduction induced by these stimuli. Furthermore, tyrosine phosphorylation of Shc in response to membrane depolarization and carbachol treatment was dependent on the presence of extracellular calcium, indicating that calcium-influx plays a role in regulation of Shc phosphorylation by these stimuli.

Similarly, PYK2 may modulate the action of ion channels that mediate their responses via and are sensitive to intracellular calcium concentration. PYK2 may therefore provide an autoregulatory role for the very same channel responsible for PYK2 activation.

The expression pattern of PYK2 and the external stimuli that activate this kinase together with its role in the control of MAP kinase signaling pathway, suggests a potential role for PYK2 in the control of a broad array of processes.

Since PYK2 activity is regulated by intracellular calcium level, both the temporal and spatial pattern of PYK2 activation may represent a carbon copy or a replica of the spatial and temporal profile of intracellular calcium concentration. Calcium concentration inside cells is highly localized because of a variety of calcium binding proteins that provide a strong buffer. Moreover, in excitable cells the level of calcium can be regulated by voltage dependent calcium channels that induce large and transient increase in intracellular calcium concentration leading to calcium oscillations and calcium waves. PYK2 may provide a mechanism for rapid and highly localized control of ion channel function, as well as, localized activation of the MAP kinase signaling pathway.

### II. Identification of Compounds that Modulate PYK2

The present invention relates, inter *alia,* to methods of detecting compounds that modulate the interaction of PYK2 with its natural binding partners. The modulation can encompass either a decrease, or an increase in the interaction between PYK2 and its natural binding partners. The compounds thus identified would be useful in the prevention or treatment of immune-related disorders involving the signal transduction system and in particular interactions among PYK2 and its natural binding partners. The compounds may be present within a complex mixture, for example, serum, body fluid, or cell extracts. Once the compounds are identified, they can be isolated using techniques well known in the art.

The present invention also encompasses a method of treating or preventing immune-related diseases in a mammal with one or more compounds, that modulate PYK2:natural binding partner interactions, comprising administering the compounds to a mammal in an amount sufficient to modulate PYK2:natural binding partner interactions.

In an effort to discover novel treatments for diseases, biomedical researchers and chemists have designed, synthesized, and tested molecules that inhibit the function of protein kinases. Some small organic molecules form a class of compounds that modulate the function of protein kinases. Examples of molecules that have been reported to inhibit the function of protein kinases include, but are not limited to, bis monocyclic, bicyclic or heterocyclic aryl compounds (PCT WO 92/20642, published November 26, 1992 by Maguire et al.), vinylene-azaindole derivatives (PCT WO 94/14808, published July 7, 1994 by Ballinari *et al*.), 1-cyclopropyl-4-pyridyl-quinolones (U.S. Patent No. 5,330,992), styryl compounds (U.S. Patent No. 5,217,999), styryl-substituted pyridyl compounds (U.S. Patent No. 5,302,606), certain quinazoline derivatives (EP Application No. 0 566 266 A1), selenoindoles and selenides (PCT WO 94/03427, published February 17, 1994 by Denny *et al*.), tricyclic polyhydroxylic compounds (PCT WO 92/21660, published December 10, 1992 by Dow), and benzylphosphonic acid compounds (PCT WO 91/15495, published October 17, 1991 by Dow *et al*), all of which are hereby incorporated by reference herein including any figures, drawings, or tables.

Compounds that can traverse cell membranes and are resistant to acid hydrolysis are potentially advantageous as therapeutics as they can become highly bioavailable after being administered orally to patients. However, many of these protein kinase inhibitors only weakly inhibit the function of protein kinases. In addition, many inhibit a variety of protein kinases and will therefore cause multiple side-effects as therapeutics for diseases.

Some indolinone compounds, however, form classes of acid resistant and membrane permeable organic molecules. WO 96/22976 (published August 1, 1996 by Ballinari *et al*.; hereby incorporated by reference herein including any figures, drawings, or tables) describes hydrosoluble indolinone compounds that harbor tetralin, naphthalene, quinoline, and indole substituents fused to the oxindole ring. These bicyclic substituents are in turn substituted with polar moieties including hydroxylated alkyl, phosphate, and ether moieties. PCT Publication WO 98/07695, published March 26, 1998 by Tang *et al*. (Lyon & Lyon Docket No. 221/187 PCT), PCT Publication WO 96/40116, published December 19, 1996 by Tang *et al.* (Lyon & Lyon Docket No. 223/298), and International Patent Publication WO 96/22976, published August 1, 1996 by Ballinari et al., all of which are incorporated herein by reference in their entirety, including any drawings, figures, or tables describe indolinone chemical libraries of indolinone compounds harboring other bicyclic moieties as well as monocyclic moieties fused to the oxindole ring. They also teach methods of indolinone synthesis, methods of testing the biological activity of indolinone compounds in cells, and inhibition patterns of indolinone derivatives.

Other examples of substances capable of modulating kinase activity include, but are not limited to, tyrphostins, quinazolines, quinoxolines, and quinolines. The quinazolines, tyrphostins, quinolines, and quinoxolines referred to above include well known compounds such as those described in the literature. For example, representative publications describing quinazolines include Barker et al., EPO Publication No.0 520 722 A1; Jones et al., U.S. Patent No. 4,447,608; Kabbe et al., U.S. Patent No. 4,757,072; Kaul and Vougioukas, U.S. Patent No. 5, 316,553; Kreighbaum and Comer, U.S. Patent No. 4,343,940; Pegg and Wardleworth, EPO Publication No. 0 562 734 A1; Barker, et al. (1991) *Proc*. *of Am. Assoc. for Cancer Research* 32, 327; Bertino, J.R. (1979) *Cancer Research* 3, 293-304; Bertino, J.R.(1979) *Cancer Research* 9(2 part 1)293-304; Curtin, et al.(1986) *Br. J*. *Cancer* 53, 361-368; Fernandes, *et al*.(1983) *Cancer Research* 43, 1117-1123; Ferris, et al. *J*. *Org. Chem.* 44(2), 173-178; Fry, et al. (1994) *Science* 265, 1093-1095; Jackman, et al. (1981) *Cancer Research* 51, 5579-5586; Jones, *et al*. *J*. *Med. Chem.* 29 (6), 1114-1118; Lee and Skibo (1987) *Biochemistry* 26(23), 7355-7362; Lemus, *et al*. (1989) *J*. *Org. Chem*. 54, 3511-3518; Ley and Seng,(1975) Synthesis 1975, 415-522; Maxwell, *et al*. (1991) Magnetic *Resonance in Medicine* 17, 189-196; Mini, *et al*. (1985) *Cancer* Research 45, 325-330; Phillips and Castle, (1980) *J*. *Heterocyclic Chem.* 17(19), 1489-1596; Reece, *et al*. (1977) Cancer Research 47(11), 2996-2999; Sculier, et al. (1986) *Cancer* Immunol. *and Immunother.* 23, A65; Sikora, et *al*. (1984) *Cancer* Letters 23, 289-295; Sikora, *et al*. (1988) Analytical Biochem. 172, 344-355; all of which are incorporated herein by reference in their entirety, including any drawings.

Quinoxaline is described in Kaul and Vougioukas, U.S. Patent No. 5,316,553, incorporated herein by reference in its entirety, including any drawings.

Quinolines are described in Dolle, *et al*. (1994) *J. Med. Chem*. 37, 2627-2629; MaGuire (1994) *J*. *Med. Chem.* 37, 2129-2131; Burke, *et.al.* (1993) *J*. *Med*. Chem. 36, 425-432; and Burke, *et al*. (1992) *BioOrganic Med. Chem.* Letters 2, 1771-1774, all of which are incorporated by reference in their entirety, including any drawings.

Tyrphostins are described in Allen, et al.(1993) *Clin. Exp. Immunol.* 91, 141-156; Anafi, et al.(1993) *Blood* 82, 12, 3524-3529; Baker, et *al*. (1992) *J. Cell Sci.* 102, 543-555; Bilder, et al. (1991) *Amer*. *Physiol. Soc.* 6363-6143, C721-C730; Brunton, et al. (1992) *Proceedings of* Amer. *Assoc. Cancer Rsch.* 33, 558; Bryckaert, et al. (1992) *Experimental Cell Research* 199, 255-261; Dong, et al. (1993) *J*. *Leukocyte Biology* 53, 53-60; Dong, *et al*. (1993) *J*. *Immunol.* 151(5), 2717-2724; Gazit, *et al*. (1989) *J. Med. Chem.* 32, 2344-2352; Gazit, et al. (1993) *J*. *Med. Chem*. 36, 3556-3564; Kaur, *et al.* (1994) *Anti-Cancer Drugs* 5, 213-222; King, et *al*. (1991) Biochem. J. 275, 413-418; Kuo, et *al*. (1993) Cancer Letters 74, 197-202; Levitzki, A., (1992) The FASEB J. 6, 3275-3282; Lyall, et al. (1989) *J*. *Biol. Chem.* 264, 14503-14509; Peterson, et al. (1993) *The Prostate* 22, 335-345; Pillemer, et al. (1992) *Int. J. Cancer* 50, 80-85.; Posner, et *al*. (1993) Molecular Pharmacology 45, 673-683; Rendu, et *al*. (1992) *Biol. Pharmacology* 44(5), 881-888; Sauro and Thomas, (1993) Life Sciences 53, 371-376; Sauro and Thomas (1993) *J*. *Pharm.* and Experimental Therapeutics 267(3), 119-1125; Wolbring, *et al*. (1994) *J*. *Biol. Chem*. 269(36), 22470-22472; and Yoneda, et al. (1991) *Cancer Research* 51, 4430-4435; all of which are incorporated herein by reference in their entirety, including any drawings.

Other compounds that could be used as modulators include oxindolinones such as those described in U.S. patent application Serial No. 08/702,232 filed August 23, 1996 and indolinones such as those described in U.S. Patent No. 5,792,783 issued August 11, 1998, entitled "3-Heteroaryl-2-Indolinone Compounds for the Treatment of Disease" (Lyon & Lyon Docket No. 223/301, both of which are hereby incorporated herein by reference in their entirety, including any drawings, figures or tables.

### III. Pharmaceutical Formulations and Routes of Administration

The compounds described herein can be administered to a human patient *per se,* or in pharmaceutical compositions where they are mixed with other active ingredients, as in combination therapy, or suitable carriers or excipient(s). Techniques for formulation and administration of the compounds of the instant application may be found in "Remington's Pharmaceutical Sciences," Mack Publishing Co., Easton, PA, latest edition. Preferred routes include oral, transdermal, and parenteral delivery.

### a) Routes Of Administration.

Suitable routes of administration may, for example, include depot, oral, rectal, transmucosal, or intestinal administration; parenteral delivery, including intramuscular, subcutaneous, intravenous, intramedullary injections, as well as intrathecal, direct intraventricular, intraperitoneal, intranasal, or intraocular injections.

Alternately, one may administer the compound in a local rather than systemic manner, for example, via injection of the compound directly into a solid tumor, often in a depot or sustained release formulation.

Furthermore, one may administer the drug in a targeted drug delivery system, for example, in a liposome coated with tumor-specific antibody. The liposomes will be targeted to and taken up selectively by the tumor.

### b) Composition/Formulation

The pharmaceutical compositions of the present invention may be manufactured in a manner that is itself known, e.g., by means of conventional mixing, dissolving, granulating, dragee-making, levigating, emulsifying, encapsulating, entrapping or lyophilizing processes.

Pharmaceutical compositions for use in accordance with the present invention thus may be formulated in conventional manner using one or more physiologically acceptable carriers comprising excipients and auxiliaries that facilitate processing of the active compounds into preparations that can be used pharmaceutically. Proper formulation is dependent upon the route of administration chosen.

For injection, the agents of the invention may be formulated in aqueous solutions, preferably in physiologically compatible buffers such as Hanks's solution, Ringer's solution, or physiological saline buffer. For transmucosal administration, penetrants appropriate to the barrier to be permeated are used in the formulation. Such penetrants are generally known in the art.

For oral administration, the compounds can be formulated readily by combining the active compounds with pharmaceutically acceptable carriers well known in the art. Such carriers enable the compounds of the invention to be formulated as tablets, pills, dragees, capsules, liquids, gels, syrups, slurries, suspensions and the like, for oral ingestion by a patient to be treated. Pharmaceutical preparations for oral use can be obtained by adding a solid excipient, optionally grinding a resulting mixture, and processing the mixture of granules, after adding suitable auxiliaries, if desired, to obtain tablets or dragee cores. Suitable excipients are, in particular, fillers such as sugars, including lactose, sucrose, mannitol, or sorbitol; cellulose preparations such as, for example, maize starch, wheat starch, rice starch, potato starch, gelatin, gum tragacanth, methyl cellulose, hydroxypropylmethyl-cellulose, sodium carboxymethylcellulose, and/or polyvinylpyrrolidone (PVP). If desired, disintegrating agents may be added, such as the cross-linked polyvinyl pyrrolidone, agar, or alginic acid or a salt thereof such as sodium alginate.

Dragee cores are provided with suitable coatings. For this purpose, concentrated sugar solutions may be used, which may optionally contain gum arabic, talc, polyvinyl pyrrolidone, carbopol gel, polyethylene glycol, and/or titanium dioxide, lacquer solutions, and suitable organic solvents or solvent mixtures. Dyestuffs or pigments may be added to the tablets or dragee coatings for identification or to characterize different combinations of active compound doses.

Pharmaceutical preparations that can be used orally include push-fit capsules made of gelatin, as well as soft, sealed capsules made of gelatin and a plasticizer, such as glycerol or sorbitol. The push-fit capsules can contain the active ingredients in admixture with filler such as lactose, binders such as starches, and/or lubricants such as talc or magnesium stearate and, optionally, stabilizers. In soft capsules, the active compounds may be dissolved or suspended in suitable liquids, such as fatty oils, liquid paraffin, or liquid polyethylene glycols. In addition, stabilizers may be added. All formulations for oral administration should be in dosages suitable for such administration.

For buccal administration, the compositions may take the form of tablets or lozenges formulated in conventional manner.

For administration by inhalation, the compounds for use according to the present invention are conveniently delivered in the form of an aerosol spray presentation from pressurized packs or a nebuliser, with the use of a suitable propellant, e.g., dichlorodifluoromethane, trichlorofluoromethane, dichlorotetrafluoroethane, carbon dioxide or other suitable gas. In the case of a pressurized aerosol the dosage unit may be determined by providing a valve to deliver a metered amount. Capsules and cartridges of e.g. gelatin for use in an inhaler or insufflator may be formulated containing a powder mix of the compound and a suitable powder base such as lactose or starch.

The compounds may be formulated for parenteral administration by injection, *e.g.*, by bolus injection or continuous infusion. Formulations for injection may be presented in unit dosage form, e.g., in ampules or in multi-dose containers, with an added preservative. The compositions may take such forms as suspensions, solutions or emulsions in oily or aqueous vehicles, and may contain formulatory agents such as suspending, stabilizing and/or dispersing agents.

Pharmaceutical formulations for parenteral administration include aqueous solutions of the active compounds in water-soluble form. Additionally, suspensions of the active compounds may be prepared as appropriate oily injection suspensions. Suitable lipophilic solvents or vehicles include fatty oils such as sesame oil, or synthetic fatty acid esters, such as ethyl oleate or triglycerides, or liposomes. Aqueous injection suspensions may contain substances that increase the viscosity of the suspension, such as sodium carboxymethyl cellulose, sorbitol, or dextran. Optionally, the suspension may also contain suitable stabilizers or agents that increase the solubility of the compounds to allow for the preparation of highly concentrated solutions.

Alternatively, the active ingredient may be in powder form for constitution with a suitable vehicle, *e.g.*, sterile pyrogen-free water, before use.

The compounds may also be formulated in rectal compositions such as suppositories or retention enemas, *e.g*., containing conventional suppository bases such as cocoa butter or other glycerides.

In addition to the formulations described previously, the compounds may also be formulated as a depot preparation. Such long acting formulations may be administered by implantation (for example subcutaneously or intramuscularly) or by intramuscular injection. Thus, for example, the compounds may be formulated with suitable polymeric or hydrophobic materials (for example as an emulsion in an acceptable oil) or ion exchange resins, or as sparingly soluble derivatives, for example, as a sparingly soluble salt.

A pharmaceutical carrier for the hydrophobic compounds of the invention is a cosolvent system comprising benzyl alcohol, a nonpolar surfactant, a water-miscible organic polymer, and an aqueous phase. The cosolvent system may be the VPD co-solvent system. VPD is a solution of 3% w/v benzyl alcohol, 8% w/v of the nonpolar surfactant polysorbate 80, and 65% w/v polyethylene glycol 300, made up to volume in absolute ethanol. The VPD co-solvent system (VPD:D5W) consists of VPD diluted 1:1 with a 5% dextrose in water solution. This co-solvent system dissolves hydrophobic compounds well, and itself produces low toxicity upon systemic administration. Naturally, the proportions of a co-solvent system may be varied considerably without destroying its solubility and toxicity characteristics. Furthermore, the identity of the co-solvent components may be varied: for example, other low-toxicity nonpolar surfactants may be used instead of polysorbate 80; the fraction size of polyethylene glycol may be varied; other biocompatible polymers may replace polyethylene glycol, e.g. polyvinyl pyrrolidone; and other sugars or polysaccharides may substitute for dextrose.

Alternatively, other delivery systems for hydrophobic pharmaceutical compounds may be employed. Liposomes and emulsions are well known examples of delivery vehicles or carriers for hydrophobic drugs. Certain organic solvents such as dimethylsulfoxide also may be employed, although usually at the cost of greater toxicity. Additionally, the compounds may be delivered using a sustained-release system, such as semipermeable matrices of solid hydrophobic polymers containing the therapeutic agent. Various types of sustained-release materials have been established and are well known by those skilled in the art. Sustained-release capsules may, depending on their chemical nature, release the compounds for a few weeks up to over 100 days. Depending on the chemical nature and the biological stability of the therapeutic reagent, additional strategies for protein stabilization may be employed.

The pharmaceutical compositions also may comprise suitable solid or gel phase carriers or excipients. Examples of such carriers or excipients include but are not limited to calcium carbonate, calcium phosphate, various sugars, starches, cellulose derivatives, gelatin, and polymers such as polyethylene glycols.

Many of the PTK modulating compounds of the invention may be provided as salts with pharmaceutically compatible counterions. Pharmaceutically compatible salts may be formed with many acids, including but not limited to hydrochloric, sulfuric, acetic, lactic, tartaric, malic, succinic, etc. Salts tend to be more soluble in aqueous or other protonic solvents that are the corresponding free base forms.

### c) Effective Dosage.

Pharmaceutical compositions suitable for use in the present invention include compositions where the active ingredients are contained in an amount effective to achieve its intended purpose. More specifically, a therapeutically effective amount means an amount of compound effective to prevent, alleviate or ameliorate symptoms of disease or prolong the survival of the subject being treated. Determination of a therapeutically effective amount is well within the capability of those skilled in the art, especially in light of the detailed disclosure provided herein.

For any compound used in the methods of the invention, the therapeutically effective dose can be estimated initially from cell culture assays. For example, a dose can be formulated in animal models to achieve a circulating concentration range that includes the IC₅₀ as determined in cell culture (*i.e*., the concentration of the test compound which achieves a half-maximal inhibition of the PTK activity). Such information can be used to more accurately determine useful doses in humans.

Toxicity and therapeutic efficacy of the compounds described herein can be determined by standard pharmaceutical procedures in cell cultures or experimental animals, e.g., for determining the LD₅₀ (the dose lethal to 50% of the population) and the ED₅₀ (the dose therapeutically effective in 50% of the population). The dose ratio between toxic and therapeutic effects is the therapeutic index and it can be expressed as the ratio between LD₅₀ and ED₅₀. Compounds which exhibit high therapeutic indices are preferred. The data obtained from these cell culture assays and animal studies can be used in formulating a range of dosage for use in humans. The dosage of such compounds lies preferably within a range of circulating concentrations that include the ED₅₀ with little or no toxicity. The dosage may vary within this range depending upon the dosage form employed and the route of administration utilized. The exact formulation, route of administration and dosage can be chosen by the individual physician in view of the patient's condition. (See *e.g*., Fingl, *et al*. (1975) *The Pharmacological Basis of Therapeutics* Ch. 1 p.1).

Dosage amount and interval may be adjusted individually to provide plasma levels of the active moiety which are sufficient to maintain the kinase modulating effects, or minimal effective concentration (MEC). The MEC will vary for each compound but can be estimated from *in vitro* data; *e.g*., the concentration necessary to achieve 50-90% inhibition of the kinase using the assays described herein. Dosages necessary to achieve the MEC will depend on individual characteristics and route of administration. However, HPLC assays or bioassays can be used to determine plasma concentrations.

Dosage intervals can also be determined using MEC value. Compounds should be administered using a regimen that maintains plasma levels above the MEC for 10-90% of the time, preferably between 30-90% and most preferably between 50-90%.

In cases of local administration or selective uptake, the effective local concentration of the drug may not be related to plasma concentration.

The amount of composition administered will, of course, be dependent on the subject being treated, on the subject's weight, the severity of the affliction, the manner of administration and the judgment of the prescribing physician.

### d) Packaging

The compositions may, if desired, be presented in a pack or dispenser device which may contain one or more unit dosage forms containing the active ingredient. The pack may for example comprise metal or plastic foil, such as a blister pack. The pack or dispenser device may be accompanied by instructions for administration. The pack or dispenser may also be accompanied with a notice associated with the container in a form prescribed by a governmental agency regulating the manufacture, use, or sale of pharmaceuticals, which notice is reflective of approval by the agency of the form of the compound for human or veterinary administration. Such notice, for example, may be the labeling approved by the U.S. Food and Drug Administration or other government agency for prescription drugs, or the approved product insert. Compositions comprising a compound of the invention formulated in a compatible pharmaceutical carrier may also be prepared, placed in an appropriate container, and labeled for treatment of an indicated condition. Suitable conditions indicated on the label may include, for example, treatment of immune-related diseases including inflammation, and the like.

### IV. Target Diseases to be Treated or Diagnosed by Methods of the Invention

Target diseases to be treated or diagnosed by the methods of the invention are generally those that have an aberrant inflammatory response. A pathologic inflammatory response may be a continuation of an acute inflammatory response or a prolonged low-grade inflammatory response, and usually causes permanent tissue damage. Macrophage and T-cell recruitment and functions, such as cytokine production, directly contribute to inflammatory pathogenesis. There are many types of diseases and disorders associated with inflammatory responses, both acute and chronic, all of which are intended to be included under specific embodiments of the present invention.

Specific diseases of interest include, but are not limited to the following:

Inflammatory bowel diseases include ulcerative colitis and Crohn's disease. The majority of cases of ulcerative colitis are mild, being limited to rectosigmoid involvement. However clinical manifestations include bloody diarrhea, mucus, fever, abdominal pain, tenesmus, and weight loss. Complications can include toxic megacolon, colonic perforation, and cancer. The level of risk of cancer is related to the extent and duration of colitis, and may be preceded by dysplasia. Intractable disease (to drug treatment), toxic megacolon, cancer and severe dysplasia may require a colectomy.

Crohn's disease is generally more serious, occurring in any part of the GI tract with transmural inflammation, bowel wall thickening, linear ulcerations, granulomas, fissures and fistulas. Clinical manifestations include fever, abdominal pain, diarrhea, fatigue, weight loss, acute ilietis, anorectal fissures, fistulas, and abscesses. Complications may include intestinal obstruction; intestinal fistulas, and intestinal malignancy. Treatments include parenteral nutrition as well as pharmaceuticals including corticosteroids, immunosuppressive agents, and metronidazole. Surgery may be required for fixed obstruction, abscesses, persistent symptomatic fistulas, and intractability.

Connective tissue diseases involve inflammation of the connective tissue as well as altered patterns of immunoregulation.

Rheumatoid arthritis is a serious health care problem. Progressive arthritic conditions in humans cause severe pain, loss of joint mobility and disfigurement, and an overall reduction in the quality of life. In rheumatoid arthritis, the synovium hyperproliferates (aided by new blood vessels) and invades the cartilage which is destroyed. Conventional treatment for rheumatoid arthritis includes non-steroidal anti-inflammatory drugs (NSAIDs). A need exists for an effective treatment for rheumatoid arthritis that will disrupt disease progression in addition to suppression or amelioration of symptoms.

Clinical manifestations of systemic lupus erythematosus include fatigue, fever, malaise, weight loss, skin rashes (malar "butterfly" rash), photosensitivity, arthritis, myositis, oral ulcers, vasculitis, alopecia, anemia, neutropenia, thrombocytopenia, lymphadenopathy, splenomegaly, organic brain syndromes, seizures, psychosis, pleuritis, pericarditis, myocarditis, pneumonitis, nephritis, venous or arterial thrombosis, mesenteric vasculitis, and sicca syndrome. There is currently no cure. treatment is directed at controlling generalized inflammation. Drugs include salicylates and NSAIDs. New, effective drugs are desperately needed.

Clinical manifestations of progressive systemic sclerosis include Raynaud's phenomenon, scleroderma, telangiectasis, calcinosis, esophageal hypomotility, arthralgias and/or arthritis, intestinal hypofunction, pulmonary fibrosis, hypertension, and renal failure. Renal failure is the leading cause of death. There is no definitive therapy.

Clinical manifestations of the mixed connective tissue disease include Raynaud's phenomenon, polyarthritis, sclerodactyly, esophageal dysfunction, pulmonary fibrosis, and inflammatory myopathy. treatment is directed to controlling the inflammatory process in general.

Clinical manifestations of Sjogren's syndrome include xerostomia and keratoconjunctivitis sicca, nephritis, vasculitis, polyneuropathy, interstitial pneumonitis, pseudolymphoma, autoimmune thyroid disease, and congenital cardiac conduction defects in women with SSA antibodies. Treatment includes symptomatic relief of dryness as well as treatments associated with autoimmune phenomena.

Although the primary target diseases to be treated or diagnosed by the methods of the invention are those that have an aberrant inflammatory response, other diseases that involve alterations in macrophage or macrophage-like cell function (i.e. osteoclast) are also intended to be included. Examples of diseases that are not considered to be classic inflammatory response-mediated diseases include, but are not limited to, osteoarthritis, osteoperosis, osteopetrosis and atherosclerosis.

### V. Other Embodiments

Methods for evaluation of disorders, methods for monitoring changes in cells, methods of identifying compounds, methods of isolating compounds which interact with a PTK, compositions of compounds that interact with a PTK, and derivatives of complexes are disclosed in detail with respect PYK-2 in PCT publication WO 96/18738 and US Patent No. 5,837,815. These publications are hereby incorporated herein by reference in their entirety, including any drawings, tables, or figures. Those skilled in the art will appreciate that such descriptions are applicable to the present invention and can be easily adapted to it. Those skilled in the art will also appreciate that any modifications made to a complex can be manifested in a modification to any of the molecules in that complex. Thus, the invention includes any modifications to nucleic acid molecules, polypeptides, antibodies, or compounds in a complex.

In addition, the WO 96/18738 provides disclosure describing the recombinant DNA techniques pertaining to the present invention, nucleic acid vectors, the nucleic acid elements of these vectors, the types of cells that can harbor these vectors, methods of delivering these vectors to cells or tissues, methods of producing and purifying antibodies, methods of constructing hybridomas that produce these antibodies, methods of detecting signaling molecule complexes, methods of detecting interactions with natural binding partners, antibodies to complexes, disruption of PTK protein complexes, purification and production of complexes, transgenic animals containing nucleic acid vectors encoding a PTK, antisense and ribozyme approaches, and gene therapy techniques. Those skilled in the art will readily appreciate that such descriptions are applicable to the present invention and can be easily adapted to it.

Other methods associated with the invention are described in the examples disclosed herein.

### EXAMPLES

The examples below are non-limiting and are merely representative of various aspects and features of the procedures used to demonstrate the role of PYK-2 in signal transduction in inflammation.

### Materials and Methods

### Chemicals

Bradykinin, pertusis toxin, cholera toxin, forskolin, phorbol 12-myristate 13-acetate (PMA), calcium ionophore A23187, carbachol, muscarine, atrophine, mecamylamine, and 1,1-dimethyl-4-phenyl piperazinium iodide (DMPP) were purchased from Sigma.

### Cells and cell culture

PC12-rat pheochromocytoma cells were cultured in Dulbecco's modified Eagle's medium containing 10% horse serum, 5% fetal bovine serum, 100 pg/mL streptomycin and 100 units of penicillin/mL. NIH3T3, 293, GP+E-86 and PA317 cells were grown in Dulbecco's modified Eagle's medium containing 10% fetal bovine serum, 100 µg/mL streptomycin and 100 units of penicillin/mL.

### Antibodies

Antibodies against PYK2 were raised in rabbits immunized (HTI) either with a GST fusion protein containing residues 362-647 of PYK2 or with a synthetic peptide corresponding the 15 amino acids at the N-terminus of PYK2. Antisera were checked by immunoprecipitation and immunoblot analysis. The specificity was confirmed either by reactivity to the related protein Fak or by competition with the antigenic or control peptides. The antibodies were found to be specific to PYK-2; they do not cross react with FAK.

### Transfections and Infections

For stable expression in PC12 cells, PYK2 was subcloned into the retroviral vector pLXSN (Miller and Rosman, Biotechniques 7:980, 1989). The construct was used to transfect GP+E-86 cells using lipofectamine reagent (GIBCO BRL). 48 hours after transfection, virus containing supernatants were collected. Pure retrovirus-containing cell-free supernatants were added to PC12 cells in the presence of polybrene (8 pg/mL, Aldrich) for 4 hours (MCB 12 491, 1992). After 24 hours, infected PC12 cells were split into growing medium containing 350 µL/mg G418. G418 resistant colonies were isolated two to three weeks later and the level of expression was determined by Western blot analysis.

Stable cell lines of NIH3T3 that overexpress PYK2 were established by cotransfection of PYK2 subcloned into pLSV together with pSV2neo utilizing lipofectamine reagent (GIBCO BRL). Following transfection, the cells were grown in Dulbecco's modified Eagle's medium containing 10% fetal bovine serum and 1 mg/mL G418. Transient transfections into 293 cells were performed by using a calcium phosphate technique, standard in the art.

### Constructs

### GST-PYK2

A DNA fragment of λ900 bp corresponding to residues 362-647 of PYK2 was amplified by PCR utilizing the following oligonucleotide primers: 5'-CGGGATCCTCATCATCCATCCTAGGAAAGA-3' (sense) and 5'-CGGGAATTCGTCGTAGTCCCAGCAGCGGGT-3' (antisense).

The PCR product was digested with *BamHI* and *EcoRI* and subcloned into pGEX3X (Pharmacia). Expression of the GST-PYK2 fusion protein was induced by 1 mM IPTG essentially as described by Smith et al., (Gene 67:31, 1988). The fusion protein was isolated by electro-elution from SDS-PAGE.

### PYK2

The full length cDNA sequence of PYK2 was subcloned into the following mammalian expression vectors: pLSV; downstream from the SV40 early promoter, pLXSN-retroviral vector; downstream from the Mo-MuLV long terminal repeat; pRK5; downstream from the CMV promoter.

### PYK2-HA

The influenza virus hemagglutinin peptide (YPYDVPDYAS) was fused to the C-terminus of PYK2 utilizing the following oligonucleotide primers in the PCR reaction: 
5'-CACAATGTCTTCAAACGCCAC-3' and 5'-
GGCTCTAGATCACGATGCGTAGTCAGGGACATCGTATGGGRACTCTGCAGGTGGGTGG.
 GCCAG-3'. The amplified fragment was digested with RsrII and Xbal and was substituted with the corresponding fragment of PYK2. The nucleotide sequence of the final construct was confirmed by DNA sequencing.

### Kinase negative mutant/Dominant-negative

In order to construct a kinase negative mutant, Lys (457) was substituted to Ala by site directed mutagenesis utilizing the 'Transformer Site-Directed Mutagenesis Kit' (Clontech). The oligonucleotide sequence was designed to create a new restriction site of Nrul. The nucleotide sequence of the mutant was confirmed by DNA sequencing. The oligonucleotide sequence that was used for mutagenesis was: 5'-CAATGTAGCTGTCGCGACCTGCAAGAA-AGAC-3' (Nrul site - , Lys-AAC-substituted to Ala-GCG).

### Immunoprecipitation and Immunoblot Analysis

Cells were lysed in lysis buffer containing 50 mM N-2-hydroxyethylpiperazine-N'-2-ethanesulferic acid (HEPES pH 7.5), 150 mM NaCl, 10% glycerol, 1% Triton X-100, 1.5 mM MgCl₂, 1 mM ethyleneglycol-bis (β-aminoethyl ether)-N,N,N'N'-tetraacetic acid (EGTA), 10 µg leupeptin per mL, 10 µg aprotinin per mL, 1 mM phenylmethylsulfonyl fluoride (PMSF), 200 µM sodium orthovanadate and 100 mM sodium fluoride. Immunoprecipitations were performed using protein A-sepharose (Pharmacia) coupled to specific antibodies. Immunoprecipitates were washed either with HNTG' solution (20 mM HEPES buffer at pH 7.5, 150 mM NaCl, 10% glycerol, 0.1% Triton X-100, 100 mM sodium fluoride, 200 µM sodium orthovanadate) or successively with H' solution (50 mM Tris-HCl pH 8, 500 mM NaCl, 0.1% SDS, 0.2%, Triton X-100, 100 mM NaF, 200 µM sodium orthovanadate) and L' solution (10 mM Tris-HCl pH 8, 0.1% Triton X-100, 100 mM NaF, 200 µM sodium orthovanadate).

The washed immunoprecipitates were incubated for 5 min with gel sample buffer at 100 °C and analyzed by sodium dodecyl sulfate polyacrylamide gel electrophoresis (SDS-PAGE). In some experiments, the gel-embedded proteins were electrophoretically transferred onto nitrocellulose. The blot was then blocked with TBS (10 mM Tris pH 7.4, 150 mM NaCl) that contained 5% low fat milk and 1% ovalbumin. Antisera or purified mAbs were then added in the same solution and incubation was carried out for 1 h at 22 °C. For detection, the filters were washed three times (5 min each wash) with TBS/0.05% Tween-20 and reacted for 45 min at room temperature with horseradish peroxidase-conjugated protein A. The enzyme was removed by washing as described above, and the filters were reacted for 1 min with a chemiluminescence reagent (ECL, Amersham) and exposed to an autoradiography film for 1-15 min.

### In vitro kinase assay

This was carried out on immunoprecipitates in 50 µL HNTG (20 mM Hepes pH 7.5, 150 mM NaCl, 20% glycerol, 0.1% Triton X-100) containing 10 mM MnCl₂ and 5 µCi or [mN-³² P]ATP for 20 min at 22 °C. The samples were washed with H', M' and, L' washing solutions, boiled for 5 min in sample buffer and separated by SDS-PAGE.

### Example I: PYK2 Knockout Experiments

The Pyk2 gene was knocked out of mice by targeted disruption. Figure 1a shows partial restriction maps of murine Pyk2 locus, the regions used as a targeting vector, and the expected mutant allele. Solid boxes indicate exons. The probe was used for Southern blot hybridization analysis of the genomic DNA from the ES cells and tail biopsy analysis. The solid line under the Pyk2 locus marks the Pyk2 kinase domain. Arrows mark the expected *Apa1* fragments of the wild-type and mutant alleles. Southern blot hybridization analysis of the mouse tail DNA digested with *Apa1* indicates that the Pyk2 knockout was successful (Fig. 1b). Immunoblot analysis (with anti-Pyk2 antibodies) of lysates from Thymus (T), Brain (B), and Spleen (S) from wild-type, Pyk2 +/-, and Pyk2 -/- mice also demonstrated the absence of Pyk2 polypeptide in knockout mice (Fig. 1c).

### Example II: Analysis of Cytokine Production

RT-PCR (reverse transcriptase-polymerase chain reaction) was used to analyze cytokine productions from wild-type +/+ and Pyk2 -/- thioglycollate-elicited peritoneal macrophages stimulated by LPS. Thioglycollate-elicited peritoneal macrophages were incubated with or without LPS (10 µg/mL) for 3h, 6h, 12h, 24h, and 48h. Total RNAs were isolated from cells at each time point. After normalization of the amount of mRNA in the total RNAs by RT-PCR of actin mRNA, total RNAs were subjected to RT-PCR with specific probes for various cytokines.

This experiment reveals a long delay of 24 to 48 hours in the onset of production of cytokines in macrophages derived from PYK2 -/- mice (Fig. 2).

RT-PCR analysis was also used to determine the cytokine productions from wild-type +/+ and Pyk2 -/splenocytes stimulated by anti-murine CD3 antibodies. Splenocytes were incubated with or without anti-CD3 (1 µg/mL) for 12h, 24h, 48h and 72h. Total RNAs were isolated from cells at each time point. After normalization of the amount of mRNA in the total RNAs by RT-PCR of the actin mRNA, total RNAs were subjected to RT-PCR with specific probes for various cytokines.

This experiment reveals a delay of approximately 24 hours in the production of cytokines in splenocytes derived from PYK2 -/- mice (Fig. 3).

Thus, these experiments showed that macrophages and splenocytes from *pyk2* -/- mice produced decreased amounts of cytokines and chemokines, including IL-1α, IL-1β, IL-6, IL-10, TNF-α, GMCSF, IFN-γ, and MIP1-α. Lower production of cytokines and chemokines such as TNF-α and MIP1-α (among others) results in attenuated recruitment and decreased activation of macrophages, T-cells and other hematopoietic and nonhematopoietic cells involved in the inflammatory response. In addition, macrophages from knockout mice had decreased motility *in vitro.*

### Example III: Carageenen Model of Inflammation

In the subcutaneous carageenen air pouch model, a subcutaneous air pouch is surgically induced on the hind flank of the mouse. The air pouch is then filled with the immunogen carageenen, a substance that induces an inflammatory response. After 10 hours, the number and type of cells infiltrating the pouch are enumerated.

Tissues from wild type +/+ and Pyk2 -/- air pouches were treated with Carageenen for 10 hours after injection. Samples were formalin-fixed and paraffin-embedded. Sections were stained with Hematoxylin and Eosin (Fig. 4a). The number of cells and the fraction of cells infiltrating into wild type +/+ and Pyk2 -/- air pouches 10 hours after injection with Carageenen were determined.

The results show that the number of infiltrating-cells in the air pouch decreased (Fig. 4b), and that the number of macrophages decreased relative to other infiltrating cells (Fig. 4c).

### Example IV: Influenza Model of Inflammation

In the influenza model, infection with the influenza virus leads to an overwhelming pulmonary inflammatory response and eventually death.

Influenza virus-induced inflammation in mice lungs was studied in lung sections from influenza virus-infected wild-type +/+ and Pyk2 -/- mice 4 days after infection with the virus. Lungs were formalin-fixed and paraffin-embedded. Sections were stained with Hematoxylin and Eosin (Fig. 5a). Figure 5b shows a time course of the survival of mice at different doses of influenza virus.

Histologic examination of the lungs of the knockout and control mice showed that in the lungs of the *pyk2* knockout mice there was a significantly lower infiltration of inflammatory cells including PMNs (polymorphonuclear leukocytes), macrophages, and especially T-cells. Thus, on average the Pyk2 knockout mice exhibited decreased pulmonary cellular infiltrate and lived about 48 hours longer than wild-type mice after challenge with the influenza virus.

The attenuated cytokine response and migration defect *in vitro* correlate with the enhanced survival and decreased pulmonary cellular infiltrate in *pyk2* -/- mice following influenza challenge.

### Example V: Analysis of Cytokine Production in PYK2 Dominant-Negative Mutants

To evaluate the role of PYK2 in mature macrophage cells that express wild-type PYK2, kinase inactive PYK2 protein was introduced into a normal macrophage cell line. Expression of the kinase inactive PYK2 functions as a "dominant-negative" inhibitor and abrogates the function of the endogenous wild-type PYK2 protein in these macrophages.

By "dominant negative mutant protein" is meant a mutant protein that interferes with the normal PYK2 signal transduction pathway. The dominant negative mutant protein contains the domain of interest (e.g., a PYK2 polypeptide or a NBP), but has a mutation preventing proper signaling, for example by preventing binding of a second domain from the same protein. One example of a dominant negative protein is described in Millauer et al., Nature February 10, 1994. Expression of the kinase inactive PYK2 protein decreased secretion of TNF-α in response to LPS, a physiologically relevant inducer of the inflammatory response.

Tyrosine phosphorylation of Pyk2 in response to LPS stimulation was measured in P388D1 cells. Cells were starved overnight in 0.5% serum and stimulated with LPS for variable time intervals (Figure 6a). After stimulation, cells were washed, lysed, and immunoprecipitated with anti-Pyk2 antibody. Immuno-precipitates were then analyzed by western blotting. Tyrosine phosphorylated bands were detected with the anti-phosphotyrosine antibody 4G10. Maximal phosphorylation was observed after 30 minutes. Cells that had been pre-treated with either herbimycin A or genestein for 4 hours were stimulated with LPS for 30 minutes and analyzed as above (Figure 6b). Tyrosine phosphorylation was diminished by pre-treatment with non-specific inhibitors of tyrosine phosphorylation.

The murine macrophage cell line (P388D1) was transfected with doxycycline inducible plasmids in order to study the effect of a dominant-negative Pyk2 protein on macrophage function. In the first transfection, a regulator plasmid was introduced and stable, drug-resistant clones were established. These were screened by transient transfection with a doxycycline-responsive, luciferase reporter plasmid. A range of luciferase responses was obtained from different clones. Two clones were selected (based upon their doxycycline response) and transfected with response plasmids containing either HA-tagged, wild-type Pyk2 or the kinase dead Pyk2 mutant (also HA tagged). After drug selection, stable clones were screened for expression of the appropriate protein by western blotting (Figure 7).

TNF secretion in response to stimulation with either LPS (1 µg/mL) or PMA (1 µg/mL) by the doxycycline inducible clones is shown in Figure 8. Induction in the presence of doxycycline was carried out for 48 hours followed by stimulation with LPS or PMA overnight (18 hours). Cells expressing the kinase-dead Pyk2 mutant had a blunted response to either LPS or PMA compared with cells expressing the wild-type Pyk2. Secreted TNF-α was measured by ELISA. Both LPS and PMA caused secretion of TNF-α. Secretion of TNF-α was inhibited by herbimycin A and genestein, which are non-specific inhibitors of tyrosine phosphorylation.

One skilled in the art would readily appreciate that the present invention is well adapted to carry out the objects and obtain the ends and advantages mentioned, as well as those inherent therein. The molecular complexes and the methods, procedures, treatments, molecules, specific compounds described herein are presently representative of preferred embodiments are exemplary and are not intended as limitations on the scope of the invention. Changes therein and other uses will occur to those skilled in the art which are encompassed within the spirit of the invention are defined by the scope of the claims.

It will be readily apparent to one skilled in the art that varying substitutions and modifications may be made to the invention disclosed herein without departing from the scope and spirit of the invention.

All patents and publications mentioned in the specification are indicative of the levels of those skilled in the art to which the invention pertains.

The invention illustratively described herein suitably may be practiced in the absence of any element or elements, limitation or limitations which is not specifically disclosed herein. Thus, for example, in each instance herein any of the terms "comprising", "consisting essentially of" and "consisting of" may be replaced with either of the other two terms. The terms and expressions which have been employed are used as terms of description and not of limitation, and there is no intention that in the use of such terms and expressions of excluding any equivalents of the features shown and described or portions thereof, but it is recognized that various modifications are possible within the scope of the invention claimed.

In particular, although some formulations described herein have been identified by the excipients added to the formulations, the invention is meant to also cover the final formulation formed by the combination of these excipients. Specifically, the invention includes formulations in which one to all of the added excipients undergo a reaction during formulation and are no longer present in the final formulation, or are present in modified forms.

In addition, where features or aspects of the invention are described in terms of Markush groups, those skilled in the art will recognize that the invention is also thereby described in terms of any individual member or subgroup of members of the Markush group. For example, if X is described as selected from the group consisting of bromine, chlorine, and iodine, claims for X being bromine and claims for X being bromine and chlorine are fully described.

Other embodiments are within the following claims.

## Claims

1. A method for identifying compounds potentially useful to treat or to prevent a disease or disorder, wherein said disease or disorder is **characterized by** an inflammatory response involving an abnormality in a signal transduction pathway that includes an interaction between a PYK2 polypeptide and a natural binding partner, comprising assaying one or more compounds for those able to modulate said interaction as a means to identify said potentially useful compounds.

2. The method of claim 1, wherein said disease or disorder **characterized by** an inflammatory response is selected from the group consisting of inflammatory bowel diseases and connective tissue diseases.

3. The method of claim 1, wherein said one or more compounds modulate said interaction in vitro.

4. The method of claim 1, wherein said one or more compounds modulate said interaction *in vivo.*

5. The method of claim 1, wherein said one or more compounds is selected from the group consisting of tyrphostins, quinazolines, quinoxolines, quinolines, and indolinones.

6. The method of claim 5, wherein said one or more compounds is one or more indolinones.

7. The method of claim 1, wherein said interaction is selected from the group consisting of PYK2 phosphorylation, PYK2 natural binding partner phosphorylation, PYK2 de-phosphorylation, PYK2 natural binding partner de-phosphorylation, and complex formation between PYK2 and a natural binding partner.

8. A method for diagnosis of a disease or disorder, wherein said disease or disorder is **characterized by** an inflammatory response involving an abnormality in a signal transduction pathway that includes an interaction between a PYK2 polypeptide and a natural binding partner, comprising detecting a change in said interaction as an indication of said disease or disorder.

9. The method of claim 8, wherein said disease or disorder **characterized by** an inflammatory response is selected from the group consisting of inflammatory bowel diseases and connective tissue diseases.

10. The method of claim 9, wherein said inflammatory bowel diseases are selected from the group consisting of ulcerative colitis and Crohn's Disease.

11. The method of claim 9, wherein said connective tissue diseases are selected from the group consisting of rheumatoid arthritis, systemic lupus erythematosus, progressive systemic sclerosis, mixed connective tissue disease, and Sjogren's syndrome.

12. The method of claim 8, wherein said interaction is selected from the group consisting of PYK2 phosphorylation, PYK2 natural binding partner phosphorylation, PYK2 de-phosphorylation, PYK2 natural binding partner de-phosphorylation, and complex formation between PYK2 and a natural binding partner.

13. The method of claim 8, wherein said change is an increase or a decrease in said interaction.

14. A method for treating or preventing a disease or disorder, wherein said disease or disorder is **characterized by** an inflammatory response involving an abnormality in a signal transduction pathway that includes an interaction between a PYK2 polypeptide and a natural binding partner, comprising administering to a patient in need of such treatment one or more compounds, wherein said one or more compounds modulate said interaction.

15. The method of claim 14, wherein said disease or disorder **characterized by** an inflammatory response is selected from the group consisting of inflammatory bowel diseases and connective tissue diseases.

16. The method of claim 15, wherein said inflammatory bowel diseases are selected from the group consisting of ulcerative colitis and Crohn's Disease.

17. The method of claim 15, wherein said connective tissue diseases are selected from the group consisting of rheumatoid arthritis, systemic lupus erythematosus, progressive systemic sclerosis, mixed connective tissue disease, and Sjogren's syndrome.

18. The method of claim 14, wherein said patient is a mammal.

19. The method of claim 18, wherein said mammal is a human.

20. The method of claim 14, wherein said one or more compounds modulate said interaction *in vitro*.

21. The method of claim 14, wherein said one or more compounds modulate said interaction *in vivo.*

22. The method of claim 14, wherein said one or more compounds is selected from the group consisting of tyrphostins, quinazolines, quinoxolines, quinolines, and indolinones.

23. The method of claim 22, wherein said one or more compounds is one or more indolinones.

24. The method of claim 14, wherein said one or more compounds is in a pharmaceutically acceptable composition.

25. The method of claim 14, wherein said interaction is selected from the group consisting of PYK2 phosphorylation, PYK2 natural binding partner phosphorylation, PYK2 de-phosphorylation, PYK2 natural binding partner de-phosphorylation, and complex formation between PYK2 and a natural binding partner.
